# EUROPEAN PATENT SPECIFICATION

(11) **EP 3 052 614 B1**
(45) Date of publication and mention of the grant of the patent: **20.12.2017**
(21) Application number: 14787113.1
(22) Date of filing: 30.09.2014
(51) Int. Cl.: C12N 5/00, C12M 3/06, C12M 1/00

(54) **METHOD OF ENRICHING OR ISOLATING A TARGET CELL POPULATION**
VERFAHREN ZUR ANREICHERUNG UND ISOLIERUNG EINER ZIELZELLPOPULATION
PROCÉDÉ D'ENRICHISSEMENT OU D'ISOLATION D'UNE POPULATION DE CELLULES CIBLES

(30) Priority: 30.09.2013 EP 13186733
(43) Date of publication of application: 10.08.2016
(73) Proprietor: Westfälische Wilhelms-Universität Münster, 48149 Münster (DE)
(72) Inventor: ROMMEL, Christina, 48149 Muenster (DE); SCHNEKENBURGER, Juergen, 48149 Muenster (DE); DIERKER, Christian, 48149 Muenster (DE)
(74) Representative: Schiweck, Weinzierl & Koch Patentanwälte Partnerschaft mbB
(86) International application number: PCT/EP2014/070850
(87) International publication number: WO 2015/044435

(56) References cited:
- WO-A1-91/11245
- WO-A1-2011/079217
- PINZANI P ET AL: "Isolation by size of epithelial tumor cells in peripheral blood of patients with breast cancer: correlation with real-time reverse transcriptase-polymerase chain reaction results and feasibility of molecular analysis by laser microdissection", HUMAN PATHOLOGY, SAUNDERS, PHILADELPHIA, PA, US, vol. 37, no. 6, 1 June 2006 (2006-06-01), pages 711-718, XP022759654, ISSN: 0046-8177, DOI: 10.1016/J.HUMPATH.2006.01.026 [retrieved on 2006-05-27]
- VONA GIOVANNA ET AL: "Isolation by size of epithelial tumor cells: A new method for the immunomorphological and molecular characterization of circulating tumor cells", AMERICAN JOURNAL OF PATHOLOGY; [10640], ELSEVIER INC, US, vol. 156, no. 1, 1 January 2000 (2000-01-01), pages 57-63, XP002186868, ISSN: 0002-9440
- SIYANG ZHENG ET AL: "3D microfilter device for viable circulating tumor cell (CTC) enrichment from blood", BIOMEDICAL MICRODEVICES, KLUWER ACADEMIC PUBLISHERS, BO, vol. 13, no. 1, 27 October 2010 (2010-10-27), pages 203-213, XP019877255, ISSN: 1572-8781, DOI: 10.1007/S10544-010-9485-3
- ALUNNI-FABBRONI M ET AL: "Circulating tumour cells in clinical practice: Methods of detection and possible characterization", METHODS : A COMPANION TO METHODS IN ENZYMOLOGY, ACADEMIC PRESS INC., NEW YORK, NY, US, vol. 50, no. 4, 1 April 2010 (2010-04-01), pages 289-297, XP026966683, ISSN: 1046-2023 [retrieved on 2010-01-29]

## Description

### FIELD OF THE INVENTION

The present invention relates to a method of enriching and/or isolating a target cell population. Generally the target cell population is enriched/isolated from a plurality of cells, for example in a body fluid, such as blood.

### BACKGROUND OF THE INVENTION

The following description is provided to assist the understanding of the reader. None of the information provided or references cited is admitted to be prior art to the present invention.

Human blood is a complex cellular mixture which is of high diagnostic potential in the clinic, mainly due to the fact that the isolation of blood is associated with low effort, pain and risk for the patient, thereby enabling diagnosing and monitoring of the progress of diseases, without major difficulties. Apart from the analysis of characteristic blood cells and the determination and monitoring of molecular biomarkers, the isolation of cellular subpopulations from blood is of high importance. This is because the direct detection of cells within blood is not possible despite of sensitive analytical methods, due to the very low number of cells in blood. Thus, an isolation and/or enrichment of cells contained in blood has to be performed, prior to the analysis of the cells. The isolation of cellular subpopulations from blood includes, for instance, the isolation of foetal cells from maternal blood. A further example is the isolation of circulating tumour cells from patients suffering from a tumorous disease.

In view of the increasing number of tumorous diseases, early diagnosis and monitoring of tumours and therapies thereof, is of particular significance for a tumour patient. In addition, detection and monitoring of recurrent tumours and metastasis is equally important and can help to save or expand a patient's life. Methods for isolating tumour cells that circulate in the blood described in the art are essentially based on one of two different concepts. These concepts either use the physical properties of the tumour cells such as their density or size, or utilize immunoreactions against specific antigens on the surface of the tumour cells in order to "label" the cells for subsequent separation or purification steps. Examples for commercially available detection systems include, for instance, OncoQuick® using density gradient centrifugation, ScreenCell® using size exclusion, or CellSearch® or AdnaTest® using immunomagnetic particles against EpCAM or cytokeratins on the surface of the tumour cells. Other assays known in the art utilize microfluidic chips, in combination with antibody-coating, or flow cytometry.

Methods using the technique of immunolabeling require a comprehensive analysis of the tumour cells, which can, however be hampered or even prevented due to the presence of an antibody used for labelling the tumour cells. In addition, such methods often demand high technical effort, and success can heavily depends on the individual person carrying out the analysis. The same applies mutatis mutandis to methods using density gradient centrifugation. Further, the use of immunomagnetic particles, microfluidic chips and flow cytometry depends on the availability of suitable antibodies. Moreover, cells which do not express the respective antigen cannot be isolated by such methods. In addition, re-identification of tumour cells artificially introduced into blood could be shown only for about 72 % of the cells, in approaches utilizing density gradient centrifugation. In other words, nearly a third of the cells are lost in these approaches. Methods using size exclusion chromatography for isolating or enriching tumour cells from blood struggle with contamination of the sample with blood cells that have a similar size as the tumour cells. Approaches using chips bear the risk of an overload of the chip by blood cells. It is also known by those skilled in the art that methods based on flow cytometry have limited sensitivity, and large sample volumes are needed in such techniques.

Other potential approaches such as the selective adhesion of tumour cells to coated and/or structured surfaces, or the use of the differing dielectric properties of tumour cells, have so far found little interest, due to a lack of a significant number successful reports. The same applies for photoacoustic flow cytometry which has been used, e.g., for the isolation of melanoma cells.

### SUMMARY OF THE INVENTION

The present invention relates to the isolation, enrichment and/or purification of a cell from a plurality of cells. Typically the cell is of a selected cell type, whereas the plurality of cells may include a variety of cell types. While conventional methods of detecting certain types of cells in a sample such as body fluid rely on the measurement of certain marker proteins or on the presence of certain DNA fragments, a method as disclosed herein allows isolating, enriching and/or purifying desired target cells. The respective cells can subsequently be analysed in more detail or be subject to further use.

In a first aspect there is provided a method of enriching, purifying and/or isolating a target cell from a plurality of cells. The method includes introducing a plurality of cells into a separation compartment. The separation compartment has an inlet and an outlet, each of which is fluidly coupled to the ambience. The plurality of cells may be introduced into the separation compartment via any opening, which may for instance be the inlet or the outlet. The separation compartment is defined by a circumferential wall, a base and a top. The base and the top are arranged at two ends of the separation compartment, which are in opposing relationship. The base of the separation compartment includes the outlet. The outlet includes a porous medium. These pores are of a size that is smaller than the size of the target cell to be enriched, purified and/or isolated. The method also includes introducing an at least essentially constant flow of a suitable liquid through the inlet of the separation compartment. The method further includes allowing an at least essentially constant flow of liquid to exit the separation compartment via the outlet. As a result the plurality of cells is exposed to a permanent flow of liquid while the filter prevents the target cell from exiting the separation compartment. The method typically also includes introducing a fluid into the separation compartment through the inlet of the separation compartment. This fluid is at least essentially immiscible with the liquid in the separation compartment that encompasses the target cell. This fluid is further of a density that is lower than the density of the respective liquid in the separation compartment. The method typically also includes allowing liquid that encompasses the target cell in the separation compartment, i.e. generally the suitable liquid that was allowed to enter the separation compartment, to exit via the outlet. As a result the volume of the liquid in the separation compartment is allowed to decrease. Thereby the target cell is enriched, purified and/or isolated.

The target cell is in some embodiments included in a target cell population. In such embodiments the method may be taken to be a method of enriching, purifying and/or isolating a target cell population from a plurality of cells. In a respective method the pores of the porous medium included in the outlet are of a size that is larger than the average cell size of cells of the target cell population to be enriched, purified and/or isolated. In some embodiments the porous medium included in the outlet is removable. In some embodiments the method of the first aspect includes removing the porous medium.

In some embodiments the method of the first aspect, where the method is being carried out in the presence of a force of gravitation, the at least essentially constant flow of liquid introduced through the inlet of the separation compartment is allowed to create a force on the target cell population that opposes the force of gravitation.

In some embodiments the method of the first aspect further includes arranging the separation compartment in such an orientation that the outlet is positioned to face, within an angle from + 80° to - 80°, upward or downward, after the plurality of cells has been introduced into the separation compartment. In some embodiments the method of the first aspect further includes arranging the separation compartment in such an orientation that the outlet is positioned to face, within an angle from + 60° to - 60°, upward or downward. An upward orientation is a position in which the outlet faces the way which is, at least essentially and in one embodiment entirely, opposite to the direction of the force of gravitation. A downward orientation is a position in which the outlet faces the way which is, at least essentially and in one embodiment entirely, in the direction of the force of gravitation. In some embodiments the method includes arranging the separation compartment in such an orientation that the outlet is positioned to face at least essentially upward or downward. In some embodiments the separation compartment is arranged in such an orientation that the outlet is positioned to face upward or downward within an angle from about + 50° to - 50°, the force of gravitation. The separation compartment is in some embodiments arranged to face upward or downward in an angle of about 75° or more, relative to the force of gravitation. In some embodiments the separation compartment is arranged to face upward in an angle of about 45° or more, relative to the force of gravitation. In some embodiments the separation compartment is arranged to face downward in an angle of about 50° or more, relative to the force of gravitation. The separation compartment is in some embodiments arranged to face upward in an angle of about 35° or more, relative to the force of gravitation. In some embodiments the separation compartment is arranged to face downward in an angle of about 35° or more, relative to the force of gravitation.

The method of the first aspect in some embodiments further includes inclining the separation compartment relative to its original orientation, after introducing the plurality of cells into a separation compartment. The separation compartment may for example be inclined by an angle in the range from + 75° to - 75°, relative to its original orientation. The separation compartment may in some embodiments be inclined by an angle of about 80° or more, relative to its original orientation. In some embodiments the separation compartment is inclined by an angle of about 45° or more, relative to its original orientation. The separation compartment may in some embodiments be inclined by an angle of about 20° or more, relative to its original orientation. In some embodiments the separation compartment is inclined by an angle of about 10° or more, relative to its original orientation.

In some embodiments of the method of the first aspect the separation compartment may be inclined by an angle in the range from + 80° to - 80°, relative to the force of gravitation. The separation compartment may for example be inclined by an angle in the range from + 90° to - 90°, relative to the force of gravitation. The separation compartment may in some embodiments be inclined by an angle of about 70° or more, relative to the force of gravitation. In some embodiments the separation compartment is inclined by an angle of about 45° or more, relative to the force of gravitation. The separation compartment may in some embodiments be inclined by an angle of about 20° or more, relative to the force of gravitation. In some embodiments the separation compartment is inclined by an angle of about 10° or more, relative to the force of gravitation.

In some embodiments of the method of the first aspect the base and the top are arranged at two ends of the separation compartment, which are at least essentially opposite to each other. In some embodiments all or a portion of the liquid that encompasses the target cell population, generally being of the suitable liquid that was allowed to enter the separation compartment, is collected after the volume of this liquid has been allowed to decrease. The liquid eluting via the outlet is collected together with the target cell population. The porous medium of the outlet is typically of solid nature. In some embodiments the outlet of the separation compartment includes a filter, which has pores.

In some embodiments the fluid introduced into the separation compartment is a gas such as nitrogen or air. In some embodiments the liquid introduced through the inlet of the separation compartment is an aqueous liquid. In some embodiments the liquid introduced through the inlet of the separation compartment is a fluorocarbon liquid.

In a second aspect there is provided a method of enriching, purifying and/or isolating a target cell from a plurality of cells. The method includes introducing a plurality of cells into a separation compartment. The separation compartment has an inlet and an outlet, each of which is fluidly coupled to the ambience. The plurality of cells may be introduced into the separation compartment via any opening, which may for instance be the inlet or the outlet. The separation compartment is defined by a circumferential wall, a base and a top. The base and the top are arranged at two ends of the separation compartment, which are in opposing relationship. The base of the separation compartment includes the outlet. The outlet includes a porous medium. These pores are of a size that is smaller than the size of the target cell to be enriched and/or isolated. The top of the separation compartment includes the inlet. The method also includes introducing an at least essentially constant flow of a suitable liquid through the inlet of the separation compartment. The method further includes allowing an at least essentially constant flow of liquid to exit the separation compartment via the outlet. As a result the plurality of cells is exposed to a permanent flow of liquid while the filter prevents the target cell from exiting the separation compartment. The method also includes arranging the separation compartment in such an orientation that the outlet faces downward. This orientation is a position in which the outlet faces the way which is, at least essentially, in the direction of the force of gravitation. The method typically also includes introducing a fluid into the separation compartment through the inlet of the separation compartment. This fluid is at least essentially immiscible with the liquid in the separation compartment that encompasses the target cell. This fluid is further of a density that is lower than the density of the respective liquid in the separation compartment. The method typically also includes allowing liquid that encompasses the target cell in the separation compartment, i.e. generally the suitable liquid that was allowed to enter the separation compartment, to exit via the outlet. As a result the volume of the liquid in the separation compartment is allowed to decrease. Thereby the target cell is enriched, purified and/or isolated.

In some embodiments of the method of the second aspect the target cell is included in a target cell population. In such embodiments the method may be taken to be a method of enriching, purifying and/or isolating a target cell population from a plurality of cells. In a respective method the pores of the porous medium included in the outlet are of a size that is smaller than the average cell size of cells of the target cell population to be enriched, purified and/or isolated. In some embodiments the porous medium included in the outlet is removable. In some embodiments the method of the first aspect includes removing the porous medium.

The method of the second aspect in some embodiments further includes arranging the separation compartment in such an orientation that the outlet is positioned to face within an angle from + 80° to - 80°, upward or downward, after the plurality of cells has been introduced into the separation compartment. In some embodiments the method includes arranging the separation compartment in such an orientation that the outlet is positioned to face at least essentially upward or downward. In some embodiments the separation compartment is arranged in such an orientation that the outlet is positioned to face upward or downward within an angle from about + 75° to - 75°, the force of gravitation. The separation compartment is in some embodiments arranged to face upward in an angle of about 75 ° or more, relative to the force of gravitation. In some embodiments the separation compartment is arranged to face downward in an angle of about 75 ° or more, relative to the force of gravitation. In some embodiments the separation compartment is arranged to face upward in an angle of about 40 ° or more, relative to the force of gravitation. In some embodiments the separation compartment is arranged to face downward in an angle of about 40 ° or more, relative to the force of gravitation. The separation compartment is in some embodiments arranged to face upward in an angle of about 20 ° or more, relative to the force of gravitation. In some embodiments the separation compartment is arranged to face downward in an angle of about 20 ° or more, relative to the force of gravitation.

The method of the second aspect in some embodiments further includes inclining the separation compartment relative to its original orientation, after introducing the plurality of cells into a separation compartment. The separation compartment may for example be inclined by an angle in the range from + 80° to - 80°, relative to its original orientation. The separation compartment may in some embodiments be inclined by an angle of about 70 ° or more, relative to its original orientation. In some embodiments the separation compartment is inclined by an angle of about 50 ° or more, relative to its original orientation. The separation compartment may in some embodiments be inclined by an angle of about 30 ° or more, relative to its original orientation. In some embodiments the separation compartment is inclined by an angle of about 15 ° or more, relative to its original orientation. The separation compartment may in some embodiments be inclined by an angle of about 5 ° or more, relative to its original orientation.

In some embodiments of the method of the second aspect the separation compartment may be inclined by an angle in the range from + 80° to - 98°, relative to the force of gravitation. The separation compartment may for example be inclined by an angle in the range from + 80° to - 80°, relative to the force of gravitation. The separation compartment may in some embodiments be inclined by an angle of about 70 ° or more, relative to the force of gravitation. In some embodiments the separation compartment is inclined by an angle of about 50 ° or more, relative to the force of gravitation. The separation compartment may in some embodiments be inclined by an angle of about 30 ° or more, relative to the force of gravitation. In some embodiments the separation compartment is inclined by an angle of about 15 ° or more, relative to the force of gravitation. The separation compartment may in some embodiments be inclined by an angle of about 5 ° or more, relative to the force of gravitation.

In some embodiments of the method of the second aspect arranging the separation compartment in such an orientation that the outlet faces downward includes rotating the separation compartment, for example by up to about 180°.

In a third aspect there is provided a method of enriching, purifying and/or isolating a target cell from a plurality of cells. The method includes introducing a plurality of cells into a separation compartment. The separation compartment has an inlet and an outlet, each of which is fluidly coupled to the ambience. The plurality of cells may be introduced into the separation compartment via any opening, which may for instance be the inlet or the outlet. The separation compartment is defined by a circumferential wall, a base and a top. The base and the top are arranged at two ends of the separation compartment, which are in opposing relationship. The base of the separation compartment includes the outlet. The outlet includes a porous medium. These pores are of a size that is smaller than the size of the target cell to be enriched and/or isolated. The inlet is included in the circumferential wall of the separation compartment. The inlet is arranged in vicinity to the outlet. The method also includes introducing an at least essentially constant flow of a suitable liquid through the inlet of the separation compartment. The method further includes allowing an at least essentially constant flow of liquid to exit the separation compartment via the outlet. As a result the plurality of cells is exposed to a permanent flow of liquid while the filter prevents the target cell from exiting the separation compartment. The method typically also includes introducing a fluid into the separation compartment through the inlet of the separation compartment. This fluid is at least essentially immiscible with the liquid in the separation compartment that encompasses the target cell. This fluid is further of a density that is lower than the density of the respective liquid in the separation compartment. Typically the method also includes allowing liquid that encompasses the target cell in the separation compartment, i.e. generally liquid of the suitable liquid that was allowed to enter the separation compartment, to exit via the outlet. As a result the volume of the liquid in the separation compartment is allowed to decrease. Thereby the target cell is enriched, purified and/or isolated.

In some embodiments the method of the third aspect includes arranging the separation compartment in such an orientation that the outlet is positioned to face within an angle from + 80° to - 80°, upward or downward, after the plurality of cells has been introduced into the separation compartment. In some embodiments the method includes arranging the separation compartment in such an orientation that the outlet is positioned to face at least essentially upward or downward. In some embodiments the separation compartment is arranged in such an orientation that the outlet is positioned to face upward within an angle from about + 70° to - 70°, the force of gravitation. In some embodiments the separation compartment is arranged in such an orientation that the outlet is positioned to face downward within an angle from about + 80° to - 80°, the force of gravitation. The separation compartment is in some embodiments arranged to face upward in an angle of about 70 ° or more, relative to the force of gravitation. In some embodiments the separation compartment is arranged to face downward in an angle of about 70 ° or more, relative to the force of gravitation. In some embodiments the separation compartment is arranged to face upward in an angle of about 25 ° or more, relative to the force of gravitation. In some embodiments the separation compartment is arranged to face downward in an angle of about 25 ° or more, relative to the force of gravitation. The separation compartment is in some embodiments arranged to face upward in an angle of about 15 ° or more, relative to the force of gravitation. In some embodiments the separation compartment is arranged to face downward in an angle of about 15 ° or more, relative to the force of gravitation.

The method of the third aspect in some embodiments further includes inclining the separation compartment relative to its original orientation, after introducing the plurality of cells into a separation compartment. The separation compartment may for example be inclined by an angle in the range from + 80° to - 80°, relative to its original orientation. The separation compartment may in some embodiments be inclined by an angle of about 60 ° or more, relative to its original orientation. In some embodiments the separation compartment is inclined by an angle of about 40 ° or more, relative to its original orientation. The separation compartment may in some embodiments be inclined by an angle of about 25 ° or more, relative to its original orientation. The separation compartment may in some embodiments be inclined by an angle of about 15 ° or more, relative to its original orientation.

In some embodiments of the method of the third aspect the separation compartment may be inclined by an angle in the range from + 80° to - 80°, relative to the force of gravitation. The separation compartment may for example be inclined by an angle in the range from + 80° to - 80°, relative to the force of gravitation. The separation compartment may in some embodiments be inclined by an angle of about 60 ° or more, relative to the force of gravitation. In some embodiments the separation compartment is inclined by an angle of about 40 ° or more, relative to the force of gravitation. The separation compartment may in some embodiments be inclined by an angle of about 30 ° or more, relative to the force of gravitation. In some embodiments the separation compartment may be inclined by an angle of about 25 ° or more, relative to the force of gravitation.

In some embodiments of the method of the third aspect the separation compartment has two inlets, which are fluidly coupled to the ambience. The first inlet is included in the circumferential wall of the separation compartment, where it is arranged in vicinity to the outlet. The at least essentially constant flow of a suitable liquid is introduced through the first inlet. In one embodiment the fluid, which is at least essentially immiscible with the liquid that encompasses the target cell, is introduced via the second inlet.

In some embodiments of the method of the third aspect the target cell is included in a target cell population. In such embodiments the method may be taken to be a method of enriching, purifying and/or isolating a target cell population from a plurality of cells. In a respective method the pores of the porous medium included in the outlet are of a size that is larger than the average cell size of cells of the target cell population to be enriched, purified and/or isolated. In some embodiments the porous medium included in the outlet is removable. In some embodiments the method of the first aspect includes removing the porous medium.

In a fourth aspect there is provided a method of enriching, purifying and/or isolating a target cell from a plurality of cells. The method is generally performed in the presence of a gravitational force. The method includes introducing a plurality of cells into a separation compartment. The separation compartment has an inlet and an outlet, each of which is fluidly coupled to the ambience. The plurality of cells may be introduced into the separation compartment via any opening, which may for instance be the inlet or the outlet. The separation compartment is defined by a circumferential wall, a base and a top. Within the separation compartment the base and the top are arranged in an at least essentially opposing relationship to each other. The base and the top are typically arranged at two ends of the separation compartment, which are in opposing relationship. The method also includes introducing an at least essentially constant flow of a suitable liquid through the inlet of the separation compartment. The method further includes allowing an at least essentially constant flow of liquid to exit the separation compartment via the outlet. Thereby the plurality of cells is suspended in a continuous flow of liquid. As a result the continuous flow of liquid prevents the target cell from sedimenting, for instance settling under the force of gravitation. The continuous flow of liquid further prevents the target cell from exiting the separation compartment via the outlet. Thereby the target cell is enriched, purified and/or isolated.

In some embodiments of the method of the fourth aspect the outlet contains a porous medium that contains pores of a size smaller than the average cell size of cells of the target cell population. In some such embodiments the method of the fourth aspect also includes introducing a fluid into the separation compartment through the inlet of the separation compartment. This fluid is at least essentially immiscible with the liquid in the separation compartment that encompasses the target cell. This fluid is further of a density that is lower than the density of the respective liquid in the separation compartment. In one embodiment the method also includes allowing liquid that encompasses the target cell in the separation compartment, i.e. generally the suitable liquid that was allowed to enter the separation compartment, to exit via the outlet. As a result the volume of the liquid in the separation compartment is allowed to decrease.

In some embodiments of the method of the fourth aspect the base of the separation compartment includes the outlet. In some embodiments the top of the separation compartment includes the outlet.

As noted above, generally the method of the fourth aspect is carried out in the presence of a gravitational force. The method may in some embodiments, after introducing the plurality of cells into the separation compartment, include arranging the separation compartment in such an orientation that the outlet is positioned to face at least essentially opposite to the direction of the gravitational force. In some embodiments of the method of the fourth aspect the inlet is arranged in the top of the separation compartment. In such embodiments introducing an at least essentially constant flow of a suitable liquid through the inlet of the separation compartment may in some embodiments be carried out after arranging the separation compartment in such an orientation that the outlet is positioned to face within an angle of ± 80° opposite to the direction of the gravitational force. In one embodiment the separation compartment is being arranged in such an orientation that the outlet is positioned to face at least essentially opposite to the direction of the gravitational force.

In some embodiments of the method of the fourth aspect the target cell is included in a target cell population. In such embodiments the method may be taken to be a method of enriching, purifying and/or isolating a target cell population from a plurality of cells.

In some embodiments of the method of the fourth aspect the separation compartment is arranged in such an orientation that the outlet is positioned to face at least essentially in the direction of the force of gravitation, before the fluid, which is at least essentially immiscible with the liquid encompassing the target cell population, is introduced into the separation compartment.

In some embodiments of the method of the fourth aspect the outlet of the separation compartment includes a porous medium. The porous medium has pores of a size smaller than the average cell size of a typical target cell or of cells of the target cell population. As a result the porous medium prevents, in addition to the continuous flow of liquid, the target cell from exiting the separation compartment when suspending the plurality of cells in a continuous flow.

As noted above, in some embodiments the method of the fourth aspect is being carried out in the presence of a gravitational force. In some respective embodiments the at least essentially constant flow of liquid introduced through the inlet of the separation compartment is allowed to create a force on the target cell population that opposes the gravitational force.

In some embodiments of the method of the fourth aspect the fluid introduced into the separation compartment is a gas such as nitrogen or air. In some embodiments the liquid introduced through the inlet of the separation compartment is an aqueous liquid. In some embodiments, where the method of the fourth aspect the liquid introduced through the inlet of the separation compartment is different from an aqueous liquid. In some embodiments the liquid introduced through the inlet of the separation compartment is a fluorocarbon liquid.

In some embodiments of the method of the fourth aspect the separation compartment is being inclined relative to its original orientation, after introducing the plurality of cells into the separation compartment.

In some embodiments where the method of the fourth aspect is being carried out in the presence of a gravitational force, the method further includes - after introducing the plurality of cells into the separation compartment - arranging the separation compartment in such an orientation that the outlet is positioned to face within an angle from + 80° to - 80° in the direction of the gravitational force. In some embodiments the method includes, after introducing the plurality of cells into the separation compartment, arranging the separation compartment in such an orientation that the outlet is positioned to face within an angle from + 80° to - 80° opposite to the direction of the gravitational force.

In some embodiments of the method of the fourth aspect the circumferential wall has a wall portion that has an at least essentially straight surface. In some embodiments the inlet is arranged proximate to the outlet. In some embodiments the inlet is included in the top of the separation compartment. In some embodiments the separation compartment has a longitudinal axis. In some embodiments the separation compartment has an at least essentially uniform maximal width along the longitudinal axis. The circumferential wall of the separation compartment may for instance have an at least essentially cylindrical shape. In some embodiments the circumferential wall defines an at least essentially tubular portion of the separation compartment. In some embodiments the circumferential wall of the separation compartment contains a portion that contains a cross-sectional area that increases along its axis.

In some embodiments of the method of the fourth aspect the separation compartment is included in a housing.

In some embodiments, where the method of the fourth aspect is being carried out in the presence of a gravitational force, the method further includes arranging the separation compartment in such an orientation that the outlet is positioned to face within an angle from + 80° to - 80° of the gravitational force, before the fluid, which is at least essentially immiscible with the liquid encompassing the target cell population, is introduced into the separation compartment. In one embodiment the separation compartment is arranged in such an orientation that the outlet is positioned to face at least essentially in the direction of the gravitational force, before the fluid, which is at least essentially immiscible with the liquid encompassing the target cell population, is introduced into the separation compartment.

In some embodiments of the method of the fourth aspect the separation compartment has a longitudinal axis, and the outlet of the separation compartment is arranged at least essentially at the longitudinal axis. As noted above, in some embodiments the separation compartment is being arranged in such an orientation that the outlet is positioned to face at least essentially in the direction or opposite to the direction of the gravitational force. In some of these embodiments arranging the separation compartment in such an orientation includes bringing the separation compartment into a position where the longitudinal axis is at least essentially parallel to the gravitational force.

In some embodiments of the method of the fourth aspect arranging the separation compartment in such an orientation that the outlet is positioned to face within an angle from + 80° to - 80° in the direction of the gravitational force or within an angle from + 80° to - 80° opposite to the direction of the gravitational force includes rotating the separation compartment, for example by essentially about 180°.

In some embodiments of the method of the fourth aspect the separation compartment includes a single inlet. In some embodiments the separation compartment includes a plurality of inlets. In some embodiments the separation compartment includes two inlets. In some embodiments the separation compartment includes two or more inlets, of which one inlet is arranged proximate to the outlet. In such embodiments introducing an at least essentially constant flow of a suitable liquid through the inlet of the separation compartment may be carried out after arranging the separation compartment in such an orientation that the outlet is positioned to face at least essentially in the direction of the gravitational force.

In some embodiments the method of the fourth aspect includes agitating the separation compartment. The separation compartment may for example be agitated the separation compartment after the separation compartment has been arranged in an orientation where the outlet is positioned to face at least essentially in the direction of the gravitational force.

In some embodiments the method of the fourth aspect further includes collecting the liquid encompassing the target cell, and/or the target cell population, together with the target cell population from the separation compartment.

In some embodiments of the method of the fourth aspect the target cell is included in a body fluid sample, an environmental sample, a cell culture sample, a bone marrow sample, a sewage sample, a food sample, a milk sample, a forensic sample, a biological molecule production sample, a protein preparation sample, a lipid preparation sample, a carbohydrate preparation sample, or any combination thereof. The body fluid sample may for example be a blood sample, a serum sample, an amniotic fluid sample, a semen sample, a lymphatic fluid sample, a cerebrospinal fluid sample, a nasopharyngeal wash sample, a sputum sample, a mouth swab sample, a throat swab sample, a nasal swab sample, a bronchoalveolar lavage sample, a bronchial secretion sample, an urine sample, or any combination of the above. The target cell may for instance be a somatic cell, a stem cell and/or a progenitor cell. In some embodiments the target cell may be a tumour cell, a foetal cell and/or an embryonic cell.

In some embodiments of the method of the fourth aspect the enrichment of the target cell, and/or the target cell population, is at least 50 fold. In some embodiments of the method of the fourth aspect the enrichment of the target cell, and/or the target cell population, is at least 75 fold. In some embodiments of the method of the fourth aspect the enrichment of the target cell, and/or the target cell population, is at least 100 fold. In some embodiments the enrichment is at least 150 fold. In some embodiments the enrichment of the target cell, and/or the target cell population, is at least or 200 fold.

In a fifth aspect there is provided a method of enriching, purifying and/or isolating a target cell from a plurality of cells. The method includes introducing a plurality of cells into a separation compartment. The separation compartment has an inlet and an outlet, each of which is fluidly coupled to the ambience. The plurality of cells may be introduced into the separation compartment via any opening, which may for instance be the inlet or the outlet. The separation compartment is defined by a circumferential wall, a base and a top. Within the separation compartment the base and the top are arranged in an at least essentially opposing relationship with each other. The base and the top may for instance be arranged at two ends of the separation compartment, which are in opposing relationship. The base of the separation compartment includes the outlet. The outlet includes a porous medium. The pores of the porous medium are of a size that is smaller than the size of the target cell to be enriched, purified and/or isolated. The method also includes introducing an at least essentially constant flow of a suitable liquid through the inlet of the separation compartment. The method further includes allowing an at least essentially constant flow of liquid to exit the separation compartment via the outlet. Thereby the plurality of cells is suspended in a continuous flow of liquid. As a result the continuous flow of liquid prevents the target cell from sedimenting, for instance settling under the force of gravitation. The porous medium that is included in the outlet prevents the target cell from exiting the separation compartment via the outlet. Thereby the target cell is enriched, purified and/or isolated.

As noted above, the porous medium included in the outlet of the separation compartment prevents, in addition to the continuous flow of liquid, the target cell from exiting the separation compartment. In some embodiments the method of the fifth aspect also includes introducing a fluid into the separation compartment through the inlet of the separation compartment. This fluid is at least essentially immiscible with the liquid in the separation compartment that encompasses the target cell. This fluid is further of a density that is lower than the density of the respective liquid in the separation compartment. A method according to such embodiments may also include allowing liquid that encompasses the target cell in the separation compartment, i.e. generally the suitable liquid that was allowed to enter the separation compartment, to exit via the outlet. As a result the volume of the liquid in the separation compartment is allowed to decrease.

In some embodiments of the method of the fifth aspect the separation compartment is arranged in such an orientation that the outlet is positioned to face at least essentially in the direction of the force of gravitation, before the fluid, which is at least essentially immiscible with the liquid encompassing the target cell population, is introduced into the separation compartment.

In typical embodiments the method of the fifth aspect is being carried out in the presence of a gravitational force. In some of these embodiments the method further includes arranging the separation compartment in such an orientation that the outlet is positioned to face at least essentially in the direction of the gravitational force, after introducing the plurality of cells into the separation compartment. In some embodiments where the method of the fifth aspect is being carried out in the presence of a gravitational force, the separation compartment is arranged in such an orientation that the outlet is positioned to face within an angle from + 80° to - 80° of the direction of the gravitational force, while introducing the at least essentially constant flow of a suitable liquid through the inlet of the separation compartment. In some embodiments the separation compartment is arranged in such an orientation that the outlet is positioned to face at least essentially in the direction of the gravitational force, while introducing the at least essentially constant flow of a suitable liquid through the inlet of the separation compartment.

In some embodiments of the method of the fifth aspect the target cell is included in a target cell population. In such embodiments the method may be taken to be a method of enriching, purifying and/or isolating a target cell population from a plurality of cells.

In some embodiments of the method of the fifth aspect the separation compartment is arranged in such an orientation that the outlet is positioned to face at least essentially in the direction of the force of gravitation, before the fluid, which is at least essentially immiscible with the liquid encompassing the target cell population, is introduced into the separation compartment.

As noted above, in some embodiments the method of the fifth aspect is being carried out in the presence of a gravitational force. In some respective embodiments the at least essentially constant flow of liquid introduced through the inlet of the separation compartment is allowed to create a force on the target cell population that opposes the gravitational force.

In some embodiments of the method of the fifth aspect the fluid introduced into the separation compartment is a gas such as nitrogen or air. In some embodiments the liquid introduced through the inlet of the separation compartment is an aqueous liquid. In some embodiments, where the method of the fifth aspect the liquid introduced through the inlet of the separation compartment is different from an aqueous liquid. In some embodiments the liquid introduced through the inlet of the separation compartment is a fluorocarbon liquid.

In some embodiments of the method of the fifth aspect the separation compartment is being inclined relative to its original orientation, after introducing the plurality of cells into the separation compartment.

In some embodiments where the method of the fifth aspect is being carried out in the presence of a gravitational force, the method further includes - after introducing the plurality of cells into the separation compartment - arranging the separation compartment in such an orientation that the outlet is positioned to face within an angle from + 80° to - 80° in the direction of the gravitational force. In some embodiments the method includes, after introducing the plurality of cells into the separation compartment, arranging the separation compartment in such an orientation that the outlet is positioned to face within an angle from + 80° to - 80° opposite to the direction of the gravitational force.

In some embodiments of the method of the fifth aspect the circumferential wall has a wall portion that has an at least essentially straight surface. In some embodiments the inlet is arranged proximate to the outlet. In some embodiments the inlet is included in the top of the separation compartment. In some embodiments the separation compartment has a longitudinal axis. In some embodiments the separation compartment has an at least essentially uniform maximal width along the longitudinal axis. The circumferential wall of the separation compartment may for instance have an at least essentially cylindrical shape. In some embodiments the circumferential wall defines an at least essentially tubular portion of the separation compartment. In some embodiments the circumferential wall of the separation compartment contains a portion that contains a cross-sectional area that increases along its axis.

In some embodiments of the method of the fifth aspect the separation compartment is included in a housing.

In some embodiments, where the method of the fifth aspect is being carried out in the presence of a gravitational force, the method further includes arranging the separation compartment in such an orientation that the outlet is positioned to face within an angle from + 80° to - 80° of the gravitational force, before the fluid, which is at least essentially immiscible with the liquid encompassing the target cell population, is introduced into the separation compartment. In one embodiment the separation compartment is arranged in such an orientation that the outlet is positioned to face at least essentially in the direction of the gravitational force, before the fluid, which is at least essentially immiscible with the liquid encompassing the target cell population, is introduced into the separation compartment.

In some embodiments of the method of the fifth aspect the separation compartment has a longitudinal axis, and the outlet of the separation compartment is arranged at least essentially at the longitudinal axis. As noted above, in some embodiments the separation compartment is being arranged in such an orientation that the outlet is positioned to face at least essentially in the direction or opposite to the direction of the gravitational force. In some of these embodiments arranging the separation compartment in such an orientation includes bringing the separation compartment into a position where the longitudinal axis is at least essentially parallel to the gravitational force.

In some embodiments of the method of the fifth aspect arranging the separation compartment in such an orientation that the outlet is positioned to face within an angle from + 80° to - 80° in the direction of the gravitational force or within an angle from + 80° to - 80° opposite to the direction of the gravitational force includes rotating the separation compartment, for example by essentially about 180°.

In some embodiments of the method of the fifth aspect the separation compartment includes a single inlet. In some embodiments the separation compartment includes a plurality of inlets. In some embodiments the separation compartment includes two inlets. In some embodiments the separation compartment includes two or more inlets, of which one inlet is arranged proximate to the outlet. In such embodiments introducing an at least essentially constant flow of a suitable liquid through the inlet of the separation compartment may be carried out after arranging the separation compartment in such an orientation that the outlet is positioned to face at least essentially in the direction of the gravitational force.

In some embodiments the method of the fifth aspect includes agitating the separation compartment. The separation compartment may for example be agitated the separation compartment after the separation compartment has been arranged in an orientation where the outlet is positioned to face at least essentially in the direction of the gravitational force.

In some embodiments the method of the fifth aspect further includes collecting the liquid encompassing the target cell, and/or the target cell population, together with the target cell population from the separation compartment.

In some embodiments of the method of the fifth aspect the target cell is included in a body fluid sample, an environmental sample, a cell culture sample, a bone marrow sample, a sewage sample, a food sample, a milk sample, a forensic sample, a biological molecule production sample, a protein preparation sample, a lipid preparation sample, a carbohydrate preparation sample, or any combination thereof. The body fluid sample may for example be a blood sample, a serum sample, an amniotic fluid sample, a semen sample, a lymphatic fluid sample, a cerebrospinal fluid sample, a nasopharyngeal wash sample, a sputum sample, a mouth swab sample, a throat swab sample, a nasal swab sample, a bronchoalveolar lavage sample, a bronchial secretion sample, an urine sample, or any combination of the above. The target cell may for instance be a somatic cell, a stem cell and/or a progenitor cell. In some embodiments target cell may be a tumour cell, a foetal cell and/or an embryonic cell.

In some embodiments of the method of the fifth aspect the enrichment of the target cell, and/or the target cell population, is at least 50 fold. In some embodiments of the method of the fifth aspect the enrichment of the target cell, and/or the target cell population, is at least 75 fold. In some embodiments of the method of the fifth aspect the enrichment of the target cell, and/or the target cell population, is at least 100 fold. In some embodiments the enrichment is at least 150 fold. In some embodiments the enrichment of the target cell, and/or the target cell population, is at least or 200 fold.

The summary of the invention described above is non-limiting and other features and advantages of the invention will be apparent from the following detailed description of the invention, and from the claims.

### BRIEF DESCRIPTION OF THE DRAWINGS

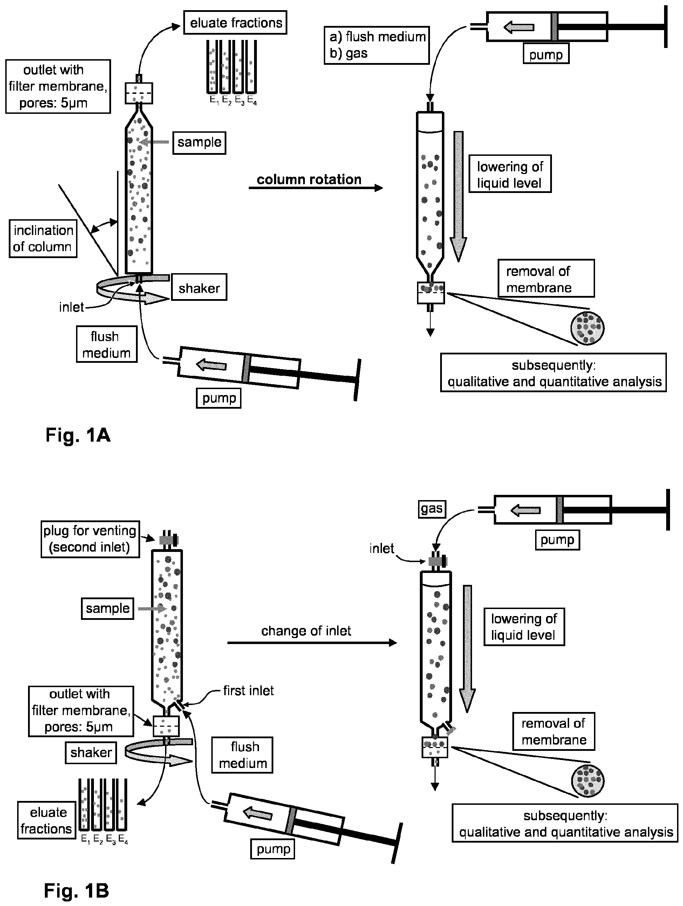

**Figure 1** illustrates two embodiments of carrying out a method of the invention. **Figure 1A** depicts an embodiment of a method of enriching a target cell population using microfiltration in combination with at least essentially linear flow. Initially the filter membrane is positioned above the column with an inlet for a feed stream at the opposite side of the column. The column is then being turned upside down and air is used as an immiscible medium to reduce the volume of liquid in the column. **Figure 1** **B** depicts an embodiment of a method of enriching a target cell population using microfiltration in combination with turbulent flow. The column is continuously kept in an orientation where the filter membrane is positioned below it. Further, the inlet for a feed stream is positioned at the same end of the column as the filter membrane. The column remains in an upright position without being inclined.

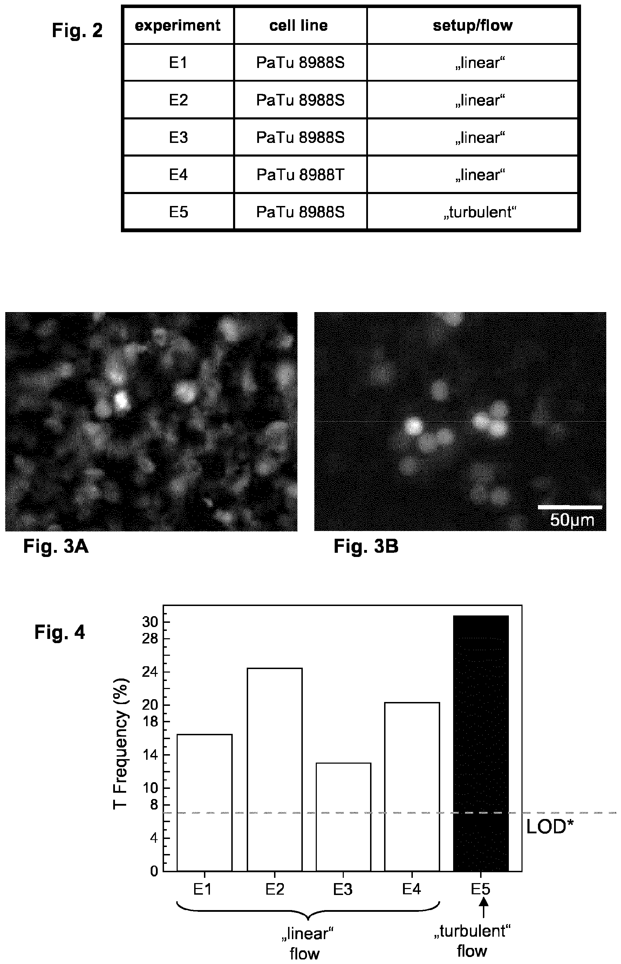

**Figure 2** is an overview of experiments carried out according to the Examples. In these experiments, to a column there were applied 5 ml of blood, into which 25.000 tumour cells had been introduced. Following cell enrichment according to a method as indicated in Fig. 1A or Fig. 1B total DNA was prepared.

**Figure 3** depicts images of tumour cells isolated according to a method of "linear" flow as illustrated in Fig. 1A (**Fig. 3A**) and according to a method of "turbulent" flow as illustrated in Fig. 1B (**Fig. 3B**). The cells were stained using CellTracker® prior to introduction. After enrichment the membrane was removed and the cells on the membrane were imaged via fluorescence microscopy.

**Figure 4** illustrates the enrichment of PaTu cells, detected by means of mutation analysis. 5 ml of blood, to which 25.000 tumour cells PaTu 8988T and PaTu 8988S had been added, were enriched in the experiments indicated in Fig. 2. Thereafter a KRAS test for a mutation in codon 12/13 was carried out in duplicate. LOD is the limit of detection as indicated by the supplier (Qiagen AG, Germany) of the pyrosequencing kit.

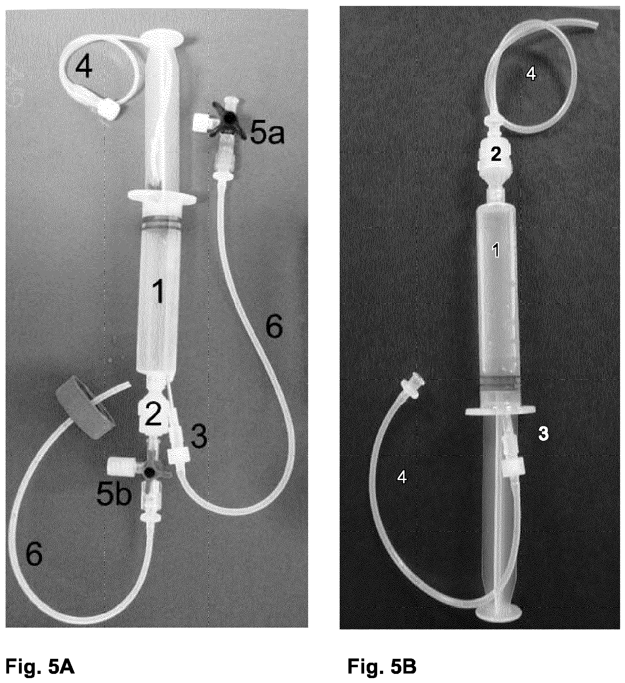

**Figure 5** shows an illustration of a filtration column assembled for downward (**Fig. 5A**) and upward (**Fig. 5B**) orientation of the outlet of the separation compartment. Fig. 5A: 1 = Original-Perfusor-Syringe OPS 20ml Luer Lock Duo 2.0x30mm; 2 = 13mm Swinnex Syringe Filter Holder with a hydrophilic Polycarbonate Membrane Filter of 5µm pore size, 13mm; 3 = Original-Perfusor hollow needle 2.0x30mm (part of 1); 4 = Venofix Luer Lock 0.8x20mm; 5a = Discofix C stopcock; 5b = 360° stopcock, 4-way, blue; 6 = flexible tubes and connecting pieces. Fig. 5B: 1 = OPS 20ml Luer Lock Duo (supra); 2 = 13mm Swinnex Syringe Filter Holder with a hydrophilic Polycarbonate Membrane Filter of 5µm pore size, 13mm (supra); 3 = Original-Perfusor hollow needle 2.0x30mm (supra); 4 = flexible tubes and connecting pieces (supra).

### DETAILED DESCRIPTION OF THE INVENTION

The present invention relates to enriching and/or isolating target cells that are included in a plurality of cells. The term "target cell" or "desired cell" or "cell of interest" as used refers to any cell that can be found in or brought into suspension. A target cell may be an epithelial cell, a mesenchymal cell, an endothelial cell, a tumour cell or a cancer cell, an infected cell, a mutated cell, a damaged cell, a stem cell, or any other cell of interest in a fluid sample as defined in the following. In some embodiments the target cell is of a specific cell type whose presence, number, proportion, or properties are useful for a diagnostic or prognostic assessment of the subject from whom the sample was collected. The subject can be an animal, such as a mammal. In some embodiments the subject is one of a rat, a mouse, a goat, a pig, a marmoset, a rabbit and an ape. In one embodiment the subject is a human. In some embodiments the target cell is a rare cell, relative to the natural environment from which it is being collected. A "rare cell" or "rare target cell" may be of a cell type that is less than about 10% or less than about 5% of the total nucleated cell population in a fluid sample as defined below. A rare cell may in some embodiments be of a cell type that is less than about 4% or less of the total nucleated cell population. In some embodiments a rare cell is of a cell type that is less than about 3%, 2% or even less than 1% of the total nucleated cell population in a fluid sample as defined below. A rare cell may also be of a cell type that is present at less than about one million cells per millilitre (ml) of fluid sample. In some embodiments a rare cell may be of a cell type that is present at less than about 500.000 cells per millilitre of fluid sample. A rare cell may in some embodiments be of a cell type that is present at less than about 100.000 cells, less than 75.000 cells, less than 50.000 cells, less than 25.000 cells, less than 10.000 cells, less than 5000 cells or even less than 1000 or 500 cells per millilitre (ml) of fluid sample. A target cell may for instance be a specific type of rare cell of interest that may be identified and/or separated and/or enriched from other types of cells by methods involving a marker or property that may be present or absent on the target cell. In some embodiments the target cell in a method disclosed herein is a tumour cell, such as a circulating tumour or cancer cell. In some embodiments the target cell is a foetal cell or an embryonic cell. In some embodiments the target cell is a stem cell.

Cells that are desirably removed, in order to facilitate identifying, isolating, purifying and/or characterizing a target cell are in the following also referred to as "undesired cells" or "non-target cells". For example, blood cells in a blood sample may be such undesired cells. A fluid sample as used herein can contain a plurality of types of non-target cells. The non-target cells can be removed by methods known in the art, e.g., by microfiltration, centrifugation, lysis, selective binding to a solid support or an antibody. Removing of non-target cells from the sample means that at least the majority of the non-target cell population is removed from the sample by the removal step, such as at least about 70% or 80% of cells that are not desired, i.e. non-target cells. Removing non-target cells from the sample may in some embodiments involve removing from the sample at least about 85%, 90%, 95%, 96%, 97%, 98%, 99%, 99.5% or even 100% of the non-target cells. Non-target cells are removed when they are taken out of the sample by the above-mentioned methods disclosed in this specification. For example, it may be desirable to remove red and white blood cells from a blood sample of a human patient suffering from a tumorous disease in order to isolate, purify and/or enrich circulating tumour cells.

The term "fluid sample" as used herein refers generally to any fluid from which cellular components are to be separated or analysed. Such a sample can be from any source, such as an environmental sample, such as from body of water or soil, an organism, a group of organisms from the same or different species, from a food source or an industrial source. Typically, the fluid sample is a biological sample, e.g., a sample of a body fluid, a sample of separated cells or a sample from a tissue or an organ. Samples of body fluids can be obtained by well-known techniques and include, but are not limited to, samples of blood, plasma, serum or urine. Tissue or organ samples may be obtained from any tissue or organ by, e.g., biopsy. In one embodiment the fluid sample is a blood sample, such as a human blood sample. A blood sample can refer to a processed or unprocessed blood sample, i.e., it may have been centrifuged, filtered, extracted, or otherwise treated, including a blood sample to which one or more reagents such as, but not limited to, anticoagulants or stabilizers have been added. An example of blood sample is a buffy coat that is obtained by processing human blood for white blood cells. Another example of a blood sample is a blood sample which has been "washed" to remove serum components by centrifuging the sample to pellet cells, removing the serum supernatant, and re-suspending the cells in a solution or buffer. Other blood samples include cord blood samples, bone marrow aspirates, internal blood or peripheral blood. A blood sample can be of any volume, and can be from any subject such as an animal, including a human. In some embodiments the fluid sample is a whole blood sample. In some embodiments the subject is a human, such as a human patient suffering from a tumour. In some embodiments the sample is a peripheral blood sample.

The plurality of cells may originate from any desired sample. The sample may be of any origin. It may for instance, but not limited to, be derived from humans, animals, plants, bacteria, viruses, spores, fungi, or protozoae, or from organic or inorganic materials of synthetic or biological origin. Accordingly, any of the following samples selected from, but not limited to, the group consisting of a soil sample, an air sample, an environmental sample, a cell culture sample, a bone marrow sample, a rainfall sample, a fallout sample, a sewage sample, a ground water sample, an abrasion sample, an archaeological sample, a food sample, a blood sample, a serum sample, a plasma sample, an urine sample, a stool sample, a semen sample, a lymphatic fluid sample, a cerebrospinal fluid sample, a nasopharyngeal wash sample, a sputum sample, a mouth swab sample, a throat swab sample, a nasal swab sample, a bronchoalveolar lavage sample, a bronchial secretion sample, a milk sample, an amniotic fluid sample, a biopsy sample, a cancer sample, a tumour sample, a tissue sample, a cell sample, a cell culture sample, a cell lysate sample, a virus culture sample, a nail sample, a hair sample, a skin sample, a forensic sample, an infection sample, a nosocomial infection sample, a production sample, a drug preparation sample, a biological molecule production sample, a protein preparation sample, a lipid preparation sample, a carbohydrate preparation sample, a space sample, an extraterrestrial sample or any combination thereof may be processed in the method. Where desired, a respective sample may have been preprocessed to any degree. As an illustrative example, a tissue sample may have been digested, homogenised or centrifuged prior to being used with the device of the present invention. The sample may furthermore have been prepared in form of a fluid, such as a solution. Examples include, but are not limited to, a solution or a slurry of a nucleotide, a polynucleotide, a nucleic acid, a peptide, a polypeptide, an amino acid, a protein, a synthetic polymer, a biochemical composition, an organic chemical composition, an inorganic chemical composition, a metal, a lipid, a carbohydrate, a combinatory chemistry product, a drug candidate molecule, a drug molecule, a drug metabolite or of any combinations thereof. Further examples include, but are not limited to, a suspension of a metal, a suspension of metal alloy, and a solution of a metal ion or any combination thereof, as well as a suspension of a cell, a virus, a microorganism, a pathogen, a radioactive compound or of any combinations thereof. It is understood that a sample may furthermore include any combination of the aforementioned examples.

A "white blood cell" as used herein means a leukocyte, or a cell of the hematopoietic lineage that is not a reticulocyte or platelet and that can be found in the blood of an animal or human. A leukocyte may for instance be a natural killer cell (NK cell) and a lymphocyte such as a B lymphocyte (B cell) or a T lymphocyte (T cell). A leukocyte can also include a phagocytic cell, such as a monocyte, a macrophage and a granulocyte, including a basophil, an eosinophil and a neutrophil. A leukocyte may also be a mast cell.

A "red blood cell" as used herein is an erythrocyte. Unless designated a nucleated red blood cell or foetal nucleated red blood cell as used herein, red blood cell is used to mean a non-nucleated red blood cell.

"Neoplastic cells" or "tumour cells" or "cancer cells" as used herein mean cells that were part of a tumour or the progeny of such cells. These cells tend to show partial or complete lack of structural organization and functional coordination with the normal tissue and may be benign or malignant. Cancer cells unlike benign tumour cells exhibit the properties of invasion and metastasis and are highly anaplastic. Cancer cells include the two broad categories of carcinoma and sarcoma.

A "tumour" or "neoplasm" is an abnormal growth of tissue resulting from uncontrolled, progressive multiplication of cells and serving no physiological function.

A "cancer" is any of various malignant neoplasms characterized by the proliferation of anaplastic cells that tend to invade surrounding tissue and metastasize to new body sites.

A "malignant cell" as used herein is a cell having the property of locally invasive and destructive growth and distal metastasis. Examples of malignant cells include, but are not limited to, leukaemia cells, lymphoma cells, cancer cells of solid tumours, metastatic solid tumour cells, e.g. breast cancer cells, prostate cancer cells, pancreas cancer cells, lung cancer cells, colon cancer cells, in various body fluids including blood, bone marrow, ascitic fluid, stool, urine bronchial washes and so on.

A "cancerous cell" as used herein means a cell that exhibits deregulated growth and, in most cases, has lost at least one of its differentiated properties, such as, but not limited to, characteristic morphology, non-migratory behaviour, cell-cell interaction and cell-signalling behaviour, protein expression and secretion pattern, etc. In most cases, cancerous cells are either of epithelial or of mesenchymal origin that are distinguishable from normal epithelial cells.

"Cancer" refers to a neoplastic disease the natural course of which is fatal. Cancer cells, unlike benign tumour cells, exhibit the properties of invasion and metastasis and are highly anaplastic. Cancer cells include the two broad categories of carcinoma and sarcoma.

A "stem cell" as used herein is an undifferentiated cell that can give rise, through one or more cell division cycles, to at least one differentiated cell type.

A "progenitor cell" is a committed but undifferentiated cell that can give rise, through one or more cell division cycles to at least one differentiated cell type. Typically, a stem cell gives rise to a progenitor cell through one or more cell divisions in response to a particular stimulus or set of stimuli, and a progenitor gives rise to one or more differentiated cell types in reply to a particular stimulus or set of stimuli.

The term "reference cell" as used herein means that the size and/or density and/or specific spatial position in the separation column and/or the concentration of the target cell is compared to the size and/or density and/or specific spatial position in the separation column and/or concentration of a reference cell. "Comparing" as used herein encompasses comparing the target cells referred to herein which are included in the fluid sample to be analysed with a suitable reference sample or reference cell. It is to be understood that comparing as used herein refers to a comparison of corresponding parameters or values, e.g., the density and/or size of a target cell as referred to herein is compared to the density and/or size of a reference cell; a concentration of a target cell as referred to herein is compared to a concentration of a reference cell; the spatial position in the separation column of the target cell is compared to the compared to the spatial position in the separation column of a reference cell; or a ratio of the concentration of two different target cells as referred to herein is compared to a ratio of two reference cells. The comparison referred to in the methods of the present invention may be carried out manually or computer assisted. For a computer assisted comparison, the value of the determined amount or ratio may be compared to values corresponding to suitable references which are stored in a database by a computer program. The computer program may further evaluate the result of the comparison by means of an expert system. Accordingly, a differential diagnosis for the diseases referred to herein may be automatically provided in a suitable output format.

The plurality of cells may be of any origin and include any desired type of cells. In some embodiments the plurality of cells is a blood sample. In one embodiment the target cells are foetal cells which are to be isolated/enriched from maternal blood. In one embodiment the target cells are tumour cells to be isolated, purified and/or enriched from blood. The invention is, at least in part, based on the surprising finding that living cells, in particular cells found in blood, in a sealed compartment can be exposed to a combination of filtration and a flow of fluid, and still most of the cells remain intact and viable. Thus cells survive undamaged and can subsequently be further used, including analysed. The use of filtration systems has so far been avoided for enriching and/or isolating target cells. The use of stirred cells for reducing the volume of a protein solution is for instance known to involve clogging of the membrane, and relative complex membrane systems are therefore used in order to avoid aggregation of proteins. Therefore the setup of stirred cells has so far been regarded as not suitable for applications that involve living cells.

By the use of the term "enriched" in reference to a cell, a polypeptide or a nucleic acid is meant that the specific cell, including cell population, or the specific amino acid/nucleotide sequence, constitutes a significantly higher fraction (2 - 5 fold) of the total number of cells or amino acid sequences or nucleic acid sequence present in the sample of interest than in the natural source from which the sample was obtained. The polypeptide, a nucleic acid or a cell may also constitute a significantly higher fraction than in a normal or diseased organism or than in normal or diseased cells or in the cells from which the sequence was taken. This could be caused by preferential reduction in the amount of other amino acid/nucleotide sequences or cells present, or by a preferential increase in the amount of the specific amino acid/ nucleotide sequence or cell of interest, or by a combination of the two. However, it should be noted that enriched does not imply that there are no other cells, amino acid sequences or nucleotide sequences present. The term merely defines that the relative amount of the sequence of interest has been significantly increased. The term significant here is used to indicate that the level of increase is useful to the person achieving such an increase, and generally means an increase relative to other amino acid or nucleic acid sequences of about at least 2-fold, for example at least about 5- to 10-fold or even more. The term is meant to cover only those situations in which man has intervened to increase the proportion of the desired amino acid sequence, nucleotide sequence or cell.

The terms "isolate" and "isolating" indicate that the cell or cells, or the peptide(s) or nucleic acid molecule(s) are being removed from its/their normal physiological environment, e.g. a natural source, or that a peptide or nucleic acid is synthesized.

The term "isolating" means taking out the desired sample component, e.g. a target cell, from a fluid sample. In the methods of the invention, a sample is enriched for the target cells of interest by an advantageous combination of several separation methods that remove other, undesired cellular components, such as blood cells in a blood sample, from the sample with high specificity. The methods of the present invention use on one side a negative or depletion approach for isolating target cells from a fluid sample. Cells such as blood cells with lower size and higher density may be washed through the porous membrane, in some embodiments at least essentially completely, and are thereby removed efficiently from the fluid sample. In contrast, the outflow of target cells from the separation column is prevented by a porous medium with specific pore size. A linear of turbulent flow can serve as an upward force for the cells which are kept at a specific spatial position within the column, in dependency from their density. "Isolating" as referred to herein also means that more than 50%, more than 60%, more than 70%, more than 80%, more than 90%, more than 95%, or even more than 96%, 97%, 98%, or 99% of the target cells in a sample are isolated by a methods described in this document. This can be determined by spiking a sample with a known number of target cells to determine how many cells are recovered for detection; as a standard, the efficiency of recovery can be determined by spiking a sample with e.g. 5, 10, 50, 100, 500 or 1000 target cells and determining how many of those cells are detected by an isolation/enrichment processes disclosed herein.

Use of the term "isolated" furthermore indicates that a naturally occurring sequence or cell has been removed from its normal environment. Thus, a sequence may be in a cell-free solution or placed in a different cellular environment. An isolated cell or isolated cells may for instance be included in a different medium such as an aqueous solution than provided originally, or placed in a different physiological environment. Typically isolated cells, peptides or nucleic acid molecule(s) constitute a higher fraction of the total cells, peptides or nucleic acid molecule(s) present in their environment, e.g. solution/suspension as applicable, than in the environment from which they were taken. By "isolated" in reference to a polypeptide or nucleic acid molecule is meant a polymer of amino acids (2 or more amino acids) or nucleotides coupled to each other, including a polypeptide or nucleic acid molecule that is isolated from a natural source or that is synthesized. The term "isolated" does not imply that the sequence is the only amino acid chain or nucleotide chain present, but that it is essentially free, e.g. about 90 - 95% pure or more, of e.g. non-amino acid material and/or non-nucleic acid material, respectively, naturally associated with it.

Isolation of a desired population of cells may in some embodiments include additional general cell enrichment techniques such as centrifugation, filtration or cell chromatography. In some embodiments isolation of a desired population of cells may also include the use of a commercially available cell isolation kit.

In some embodiments target cells that are included in a plurality of cells are purified. The term "purified" is understood to be a relative indication in comparison to the original environment of the cell, thereby representing an indication that the cell is relatively purer than in the natural environment. It therefore includes, but does not only refer to, an absolute value in the sense of absolute purity from other cells (such as a homogeneous cell population). Compared to the natural level, the level after purifying the cell will generally be at least 2-5 fold greater (e.g., in terms of cells/ml). Purification of at least one order of magnitude, such as about two or three orders, including for example about four or five orders of magnitude is expressly contemplated. It may be desired to obtain the cell at least essentially free of contamination, in particular free of other cells, at a functionally significant level, for example about 90%, about 95%, or 99% pure. With regard to a nucleic acid, a peptide or a protein, the above applies mutatis mutandis. In this case purifying the nucleic acid, peptide or protein will for instance generally be at least 2-5 fold greater (e.g., in terms of mg/ml).

"Separation" as used herein means a process in which one or more cellular components, such as a target cell and an undesired cell are spatially separated from each other or from one or more other components of a sample. A separation can be performed such that one or more sample components of interest are translocated to or retained in one or more areas of a separation apparatus such as a separation compartment, e.g. a separation column, and at least some of the remaining components are translocated away from the area or areas where the one or more sample components of interest are translocated to and/or remained in, or in which one or more sample components is retained in one or more areas and at least some of the remaining components are removed from the area or areas. Alternatively, one or more components of a sample can be translocated to and/or retained in one or more areas and one or more sample components can be removed from the area or areas. It is also possible to cause one or more sample components to be translocated to one or more areas and one or more sample components of interest or one or more components of a sample to be translocated to one or more other areas. Separation can be achieved through, for example, filtration, preferably microfiltration, or the use of physical, chemical, electrical, or magnetic forces. Non-limiting examples of forces that can be used in separations are gravity, mass flow, dielectric forces, traveling-wave dielectric forces and electro-magnetic forces.

The term "subject" as used herein, also addressed as an individual, refers to a human or non-human animal, generally a mammal. A subject may be of a mammalian species such as a rabbit, a mouse, a rat, a Guinea pig, a hamster, a dog, a cat, a pig, a cow, a goat, a sheep, a horse, a monkey, an ape or a human. Thus, the methods and uses described in this document are inter alia applicable for isolating cells found in both human and veterinary disease. A sample that includes a plurality of cells may have been obtained from a subject. It is thus understood that conclusions drawn from the presence or number of cells in the sample and decisions based thereon concern the subject from whom/which the sample has been taken. Further, while a subject is typically a living organism, a method or use described in this document may also be used in post-mortem analysis. Where the subject is a living human who is receiving medical care for a disease or condition, it is also addressed as a "patient".

A "column" or "chamber" as used herein means a structure that is capable of containing a fluid sample in which at least one processing step such as a separation step can be performed. The column or chamber can have various dimensions and its volume may vary between a few microliters and about 0.5 litres or more.

A "filter" is a structure that includes one or more pores or slots of particular dimensions that can be within a particular range that allows the passage of some sample components, e.g. cells, but not others from one side of the filter to the other, based on the size, shape, and/or deformability of the particles or cells. A filter can be made of any suitable material that prevents passage of insoluble particles, such as metal, ceramics, glass, silicon, plastics, polymers, fibres, etc. Such filters are commercially available.

The term "microfiltration" as used herein means a membrane technical filtration process which removes contaminants or cells from a fluid by passage through a microporous membrane. A typical microfiltration membrane pore size range is 0.1 to 10 micrometres (µm). Accordingly, microfiltration makes use of the same size-based separation principle. Microfiltration can use, e.g., a pressurized system. Microfiltration as used herein is the process of filtration with a micrometre sized filter. The filters can be in a submerged configuration or a pressure vessel configuration. They can be hollow fibres, flat sheet, tubular, spiral wound, hollow fine fibre or track etched. These filters are porous and allow water, monovalent species (Na⁺, Cl⁻), dissolved organic matter, small colloids, cell debris and small cells through but do not allow larger cells through.

A "filtration unit" as referred to herein is an embodiment of a separation compartment. The term refers to a filtration chamber and the associated inlets, valves, and conduits that allow sample and solutions to be introduced into the filtration chamber and sample components to be removed from the filtration chamber. A filtration unit optionally also includes a loading reservoir.

A "conduit" as used herein is a means for fluid to be transferred between different locations. Fluid may for example be transported via a conduit from a container or pump to a separation compartment such as a chamber, column or microfiltration unit. Preferably, a conduit directly or indirectly engages a port in the housing of a chamber. A conduit can comprise any material that permits the passage of a fluid through it. Conduits can comprise tubing such as for example rubber, Teflon or tygon tubing. Conduits can also be moulded out of a polymer or plastic, or drilled, etched, or machined into a metal, glass or ceramic substrate. Conduits can thus be integral to structures such as, e.g., a cartridge. A conduit can be of any dimensions but preferably ranges from 10 microns to 5 millimetres in internal diameter. A conduit is preferably enclosed, other than fluid entry and exit points, or can be open at its upper surface, as a canal-type conduit.

The term "microfiltration unit" as used herein generally refers to an embodiment of the separation compartment. The term means in a method disclosed herein using at least essentially linear flow, a microfiltration unit that includes at least (i) an inlet at the upper end, (ii) an outflow at the lower end, and (iii) a porous medium such as a membrane filter at the lower end. In the method of the invention using turbulent flow, the microfiltration unit includes (i) a porous medium such as a membrane filter, ii) an inlet, and (iii) a port which allows ventilation. Further details of a microfiltration unit and additional means which can be used in a method of the invention are indicated elsewhere herein.

A "cartridge" is a structure that includes one or more chambers that is/are part of a manual or automated system and one or more conduits for the transport of fluid into or out of at least one chamber. A cartridge may or may not include one or more chips.

An "automated system for separating target cells from a fluid sample" or an "automated system" as used herein is a device that includes one or more filtration chambers, automated means for providing fluid flow through the filtration chamber, and at least one power source for providing fluid flow and, optionally, means for providing a signal source for the generation of forces on active chips. An automated system of the present invention can also optionally include one or more active chips, separation chambers, or separation columns.

A "port" is an opening in the housing of a chamber or column through which a fluid sample such as a liquid or a gas including air can enter or exit the chamber or column. For example, a port can allow for ventilation of a microfiltration unit as used in a method disclosed herein. Also an inlet or an outlet as used herein is in some embodiments a port. A port can be of any dimensions. Typically a port is of a shape and size that allows a sample to be dispensed into a chamber by pumping a fluid through a conduit, or by means of a pipette, syringe, or other means of dispensing or transporting a sample.

The term "inlet" as used herein denotes a point of communication with the ambience at which matter such a sample, a solution, a buffer, or a reagent may be allowed to enter the separation compartment, such as a fluid column or chamber. An inlet can be a port of a chamber or column, or can be an opening in a conduit that leads, directly or indirectly, to a chamber or column of an automated system. In some embodiments, the inflow can be closed.

The term "outlet" as used herein means a point of communication with the ambience at which matter such as the sample, sample components, or reagents may be allowed to exit a fluidic chamber or column. The sample components and reagents that leave a chamber or column can be waste, i.e. sample components that are not to be used further, or can be sample components or reagents to be recovered, such as, for example, reusable reagents or eluates or fractions that include target cells to be further analysed or manipulated. An outlet can be a port of a chamber, but preferably is an opening in a conduit that, directly or indirectly, leads from a chamber of an automated system. In some embodiments, the outflow can be closed.

A "pump" as used herein can be a pressure pump or a vacuum pump. Preferably, an automated pressure pump is used in the methods of the invention. However, also a syringe can be used as a "pressure pump".

The term "shaker" as used herein means any shaker that can be used to agitate the microfiltration column as utilized in the methods of the invention. For example, laboratory shakers which are commercially available can be used to this end.

In a method of the invention typically a separation compartment is provided. The separation compartment may be included in any desired device. In some embodiments the device is selected to allow rotating or inverting the separation compartment. The separation compartment may for instance be a filtration column or a filtration chamber. In some embodiments the separation compartment is included in, placed within, a housing. Such a housing may also be adapted to take a plurality of separation compartments. Additionally, during the arrangement of a plurality of said devices, the housing may allow the plurality to be further secured together.

The separation compartment typically has a circumferential wall, a base and a top. The base and the top are arranged in opposing relationship. The term "opposing relationship" refers to the direction of matter that could flow through the separation compartment if top and base would include an inlet and an outlet, respectively. The top and the base may for example be arranged in opposing relationship on an axis of the separation compartment. Accordingly, the top and the base may be arranged in any angle with respect to each other, as long as they are not facing the same direction. The base and the top are typically arranged at at least essentially opposite ends of the separation compartment. The base and the top are in some embodiments arranged at opposing ends along a longitudinal axis of the separation compartment.

The circumferential wall is typically a straight wall. The circumferential wall has an inner surface. Likewise, the base and the top have an inner surface. The inner surface of the base is typically a straight surface. Likewise, the inner surface of the top is typically a straight surface. These inner surfaces can be taken to define the interior of the separation compartment, since they come into direct contact with fluid filled into the separation compartment. The inner surfaces also come into contact with the cells that are to be enriched, isolated or purified as well as the plurality of cells, such as a cell suspension, from which the cells are to be enriched, isolated or purified. The interior of the sample processing chamber means any space or matter that is in direct contact with fluid filled into the sample processing chamber while any inlet or outlet is closed. It also refers to any space or matter that may be included in space or matter contacting such fluid. Accordingly the term "inner wall", when used in connection with the separation compartment, refers to surface areas that face the interior of the sample processing chamber in that they are able to contact fluid filled therein while any inlet or outlet is closed. Any inlet or outlet may act to allow fluidic communication between the interior of the separation compartment and the ambience, i.e. the surrounding environment. The term "environment" refers to the exterior (vs. the interior) of the separation compartment and therefore means any matter that is not located inside or contacting the interior of the separation compartment. It thus refers to any space or matter that is not in direct contact with fluid filled into the separation compartment while any inlet or outlet is closed.

The separation compartment may include any desired material. Typically, walls of the separation compartment are solid and able to remain intact during the entire process to be performed therein. Where desired, a material included in the separation compartment is selected to withstand a fluid such as an aqueous medium or a perfluorocarbon liquid that is intended to be used. Examples of materials that are typically suitable for the use of aqueous fluids include, but are not limited to, inorganic materials and natural or synthetic polymers. Examples of suitable polymers include, but are not limited to, polypropylene, polyisoprene, polystyrene, polyvinyl chloride, polyisobutylene, polyethylene terephthalate (PET), polyacrylates (e.g. polymethyl-methacrylate (PMMA)), ethylene-vinyl acetate (EVA) copolymers, phenol formaldehyde resins, epoxy resins, poly(N-propargylamides), poly(O-propargylesters), and polysiloxanes. Two illustrative examples of inorganic materials that may be used under most conditions are glass and a metal foil. A respective metal foil may for instance be an aluminium foil. A metal foil may optionally be coated with a polymer layer such as polyethylene or polypropylene, for example were it is desired to avoid oxidation.

If desired, any part of the separation compartment, including the circumferential wall, the top and/or the bottom, may include or be of translucent material such as, glass, quartz or a plastic material. Suitable plastic materials include, but are not limited to, polymethylmethacrylates (e.g. polymethyl-methacrylate (PMMA) or carbazole based methacrylates and dimethacrylates), polystyrene, polycarbonate, and polycyclic olefins. A further illustrative example of a material that is additionally suitable for the generation of a substrate that allows light to pass only to a certain extent is fluoro-ethylene-propylene (FEP).

It is understood that the interior of the separation compartment is defined by the circumferential wall, which may be of any thickness. The separation compartment may have any desired shape, including straight, bent or meandering (or otherwise winding) and may include one or more bends or kinks. In typical embodiments the separation compartment has one longitudinal axis. The separation compartment may possess a transverse section of any desired profile, such as being a cuboid (e.g. with rectangular or square shaped profile) or alternatively a hemi-sphere or any other suitable irregular profile. The profile of the separation compartment may also change its shape along a length of the separation compartment, for instance gradually or stepwise. The circumferential wall, the base and the top may be of any geometry and dimension. They may for instance be curved, round, straight or flat. The separation compartment has in some embodiments an axis, for example a longitudinal axis.

The separation compartment may be of any desired volume. It may for example be able to accommodate a volume of about 0.1 ml to about 1000 ml, including a volume of about 1 ml to about 500 ml. In some embodiments the separation compartment may have a volume from about 10 µl to 100 µl. Where fluid is filled into the compartment, only part of the volume of the sample reaction chamber may be filled with the respective fluid. The remainder of the chamber volume may for instance be occupied by other fluid, such as air or an inert gas.

As indicated above, the separation compartment has one or more inlets and one or more outlets. Through the inlet a sample of cells may be disposed into, or enter, the separation compartment. In some embodiments a respective sample of cells, or a selection portion of the sample of cells, can also be removed from, or leave, the sample compartment via an inlet. Typically matter such as fluid is allowed to leave the separation compartment via an outlet. The inlet and the outlet may be located at any desired position relative to a selected point or region of the sample compartment. The inlet may for example be located on a top of the sample compartment. The outlet may for example be located at a bottom of the sample compartment. The terms "on top of", "on a top of", "at a bottom of", "at the bottom of", "below" and "above" as used herein refer to a position where the separation compartment is arranged in a position where an outlet of the separation compartment points into the direction of gravity. In some embodiments the outlet is arranged in the longitudinal direction of the separation compartment, i.e. in the direction that corresponds to the length of the separation compartment. In one embodiment the outlet is arranged on the longitudinal axis of the separation compartment. In some embodiments an inlet or an outlet of the sample compartment is adapted to be used as a port that allows for ventilation of the separation compartment. The port in the microfiltration allows both air intake and air outlet, i.e. the admission of air and deaeration.

The inlet and the outlet may be of any form and dimension. Examples of an inlet and an outlet include, but are not limited to, a valve, a chamber, a neck or a channel. In some embodiments the inlet is an opening. A respective opening may be of any shape, profile and diameter. The separation compartment may further include a seal for the inlet and/or a seal for the outlet. In one of the embodiments where the inlet is an opening, the separation compartment includes a seal for the respective opening. Likewise, if the outlet is an opening, the separation compartment may include a seal for the respective opening. A respective seal, which may be removable from the separation compartment, may for instance prevent matter from entering or leaving the sample compartment. A respective seal may include any desired material. Three purely illustrative examples of materials that may be used are glass, polypropylene (PP) and polytetrafluoroethylene (PFTE, Teflon). The inlet of the separation compartment may be entirely sealable.

In some embodiments the inlet is arranged in the bottom and the outlet is arranged in the top of the separation compartment. In some embodiments the arrangement of inlet and outlet allows the admission of fluid, including e.g. air, via the inlet of the separation compartment at the opposite end of the porous substrate and the outlet, which allow the outflow of fluid. For example, the separation compartment can include (i) an inlet at the upper end, (ii) an outlet at the lower end, and (iii) a membrane filter at the lower end. In some embodiments, the separation compartment can include (i) an inlet at the lower end, (ii) an outlet at the upper end, and (iii) a membrane filter at the upper end. In some embodiments the separation compartment can include (i) an inlet at the upper end, (ii) an outlet at the lower end, and (iii) a membrane filter at the lower end. As indicated above an apparatus, in which the separation compartment is included, may include further components. A respective apparatus may for instance include a pump, which is fluidly connected to the inlet, for example via a conduit. Such an arrangement permits fluid, including a gas, to be admitted to the separation compartment via the inlet. Furthermore, the separation compartment can in some embodiments be agitated in the course of the method of enriching/isolating a target cell population by a shaker that is included in a respective apparatus. As a further example, the apparatus may include one or more devices for the collection of eluates or fractions. Furthermore, a devices for carrying out any desired part of a method of using the separation compartment in an automated manner may be included in a respective apparatus.

The outlet, or in embodiments where a plurality of outlets is included in the separation compartment at least one outlet, in some embodiments includes a porous medium, which is generally of solid nature. The porous medium has pores of a defined average size. The average size of the pores is smaller than the average cell size of cells of the target cell population to be enriched, isolated and/or purified. As an illustrative example, where the cells to be isolated have an average maximal width of about 7 µm or higher, the pores of the porous medium may for instance have an average maximal width of about 5 µm. The porous medium may also have pores of a defined maximal deviation from the average size. This may be advantageous to prevent cells of the target cell population from escaping the separation compartment via particularly large pores.

In some embodiments the porous medium is a filter such as a membrane filter. The porous medium may for example be a microporous film. A respective membrane filter is commercially available, for example as a membrane disc filter. The porous medium retains particles, microorganisms or cells larger than their pore size by surface capture. In some embodiments the porous medium is of inorganic material. It may for instance include glass fibres or include silver. It may also include ceramic material. In some embodiments the porous medium is of a polymeric material. The porous medium may include cellulose, a cellulose ester, a cellulose acetate, a cellulose nitrate, polytetrafluoroethylene (PTFE), polyvinylidene fluoride (PVDF), PVC, polyethersulfone (PES), polypropylene, Nylon, or polycarbonate. In some embodiments, the porous medium as used in a method of the invention is a polycarbonate track etched (PCTE) membrane. PCTE membrane disc filters (commercially available from, e.g., Sterlitech and other manufacturers) are made from a thin, microporous polycarbonate film material. PCTE membranes are well suited for use in blood assays as well as high-purity or general filtration. A PCTE membrane is typically a thin, translucent and microporous polycarbonate film with a smooth, flat surface.

A "pore" as used herein is an opening, in particular a through-hole, in a surface, such as a membrane filter or microfilter that provides fluid communication between one side of the surface and the other. A pore can be of any size and of any shape. In some embodiments a pore is of a size and shape that restricts passage of one or more insoluble sample components from one side of a porous substrate, e.g. a filter, to the other side of the porous substrate based on the size, shape, and deformability (or lack thereof), of the sample component.

As noted above, in some embodiments the volume of the liquid encompassing the target cell population is allowed to be decreased, for example by introducing a fluid essentially immiscible with the liquid encompassing the target cell population. In such embodiments the position of the outlet, which includes the porous medium, is generally selected to be as far in the direction of gravity, relative to the entire separation compartment, that the outlet is still covered with liquid encompassing the target cell, if the volume in the separation compartment has been reduced to a desired final volume, in which the target cell is to be isolated (cf. also below). In embodiments where the base has a straight inner wall, this outlet may typically be located anywhere in this wall. In embodiments where the base has a curved inner wall, including a concave inner wall, both the position of this outlet and the exact orientation of the separation compartment may be adjusted so as to allow this outlet to be fully covered with liquid if the desired final volume of the target cell population has been reached by introducing an immiscible fluid into the separation compartment (see also below).

In some embodiments the separation compartment is adapted to allow a counter-flow mode. The term "counter-flow" as used herein means the occurrence of fluid flow in different, including opposite, directions within an apparatus such as a separation compartment. The separation compartment may be imagined as having different portions, although in fact defining a continuous space, in adjacent portions of an apparatus such as a separation compartment. This can be achieved, for instance, by positioning the inlet used for introducing a flow of liquid into the separation compartment and the outlet used to allow the flow of liquid to exit, in proximity to each other. In some embodiments, including in embodiments of counter-flow, the inflow and outflow of fluid is allowed to occur in opposed directions: The inflow of, e.g., wash buffer into the separation compartment and the outflow of, e.g., eluates or fractions from the separation compartment can take opposing directions, regardless of the arrangement of inlet and outlet. In some embodiments the respective inlet and the respective outlet used are arranged at different positions of the separation compartment, such as opposite to each other or offset to each other by a certain angle, for instance axially offset. An example of a separation compartment used in counter-flow mode is depicted, for instance, in Fig. 1B.

In a method of the invention the plurality of cells is introduced into the separation compartment. The cells may be suspended in any desired medium as long as the cells remain viable in the medium during the period of time during which the method of enriching and/or isolating a target cell population is being carried out. In some embodiments the cells are suspended in an aqueous medium. In some embodiments the cells are suspended in a fluorocarbon liquid.

In a method of enriching and/or isolating target cells as disclosed herein the separation compartment may be arranged in any desired orientation when the cells are being introduced into the separation compartment. The separation compartment is in a certain orientation when the plurality of cells has been introduced into the separation compartment. In some embodiments the orientation of the separation compartment is left unchanged. In some embodiments the separation compartment is brought into an orientation that differs from the orientation the separation compartment had when cells were being introduced. In this orientation the outlet may in some embodiments be positioned to face at least essentially in the direction of gravitation or opposite to the direction of the force of gravitation. In some embodiments the separation compartment may be inclined at an angle from about 1° to about 90°, relative to the direction of gravitation. The separation compartment may for instance be inclined at an angle from about 5° to about 85°, such as from about 10° to about 75°, relative to the direction of gravitation. In some embodiments the separation compartment is being brought into such an orientation. In some embodiments the separation compartment is already arranged in such an orientation. It may for example have been set up accordingly before or during introducing the plurality of cells.

In some embodiments, after application of the plurality of cells, for example included in a fluid sample, to the separation compartment, the separation compartment can be positioned vertically. For example, the microfiltration unit can be oriented vertically in a way that the inlet is arranged at the upper end and the outlet, including the porous medium, is arranged at the lower end. In some embodiments the microfiltration unit can be positioned vertically in a way that the inflow is at the lower end and the membrane filter and the outflow are at the upper end. In some embodiments, gravity is used to assist the process of enriching, purifying and/or isolating a target cell population if the separation compartment is positioned vertically.

Prior to application to the separation compartment, the plurality of cells can be diluted, for example, in a buffer such as a wash buffer and/or pre-treated as indicated elsewhere herein. In a method of enriching and/or isolating a target cell population, a fluid sample containing the plurality of cells, e.g., a blood sample, is loaded to the separation compartment. The outlet may be closed in order to prevent leakage of fluid sample through the outlet. In some embodiments the porous medium, also called porous substrate herein, included in the outlet may be removed temporarily for the loading process. After application of the fluid sample to the microfiltration unit, the microfiltration unit may be filled up with a liquid such as a wash buffer, for example with a port such as an inlet opened for ventilation. The separation compartment may then be brought into an upright, i.e. vertical position.

In a method disclosed herein an at least essentially constant flow of a suitable liquid is allowed to enter the separation compartment and to flow through the separation compartment. The liquid is allowed to enter the separation compartment via an inlet and to exit the separation compartment via an outlet (supra). A turbulent flow of liquid, such as a turbulent or an at least essentially linear flow of wash buffer, may be applied via an inlet of the separation compartment. The total amount of liquid that is allowed to pass through the separation compartment is selected according to the individual conditions of the respective embodiment, in particular the dimensions of the separation compartment, and the amount of cells present in the plurality of cells. In some embodiments a volume in the range from about 100 ml to about 2 litres of liquid is used. Likewise, the flow rate is selected according to the individual conditions of the respective embodiment, such as the pore size of the porous medium, the dimensions of the outlet and of the porous medium, and the volume of the separation compartment. In some embodiments a flow rate in the range from about 20 ml/h to about 500 ml/hr of liquid is used.

"Turbulent flow" as used herein means that fluid such as a wash buffer is pumped or injected into a separation compartment in a laminar or turbulent way during the separation process. As known to those skilled in the art, turbulent flow is a type of fluid flow in which the fluid undergoes irregular fluctuations, or mixing, in contrast to laminar or linear flow, in which the fluid moves in smooth paths or layers. In turbulent flow, the speed of the fluid at a point is continuously undergoing changes in both magnitude and direction.

"Linear flow" as used herein means that fluid such as a wash buffer is pumped or injected into a separation compartment such as a chamber, column or microfiltration unit continuously and constantly during the separation process. This allows for components of a sample that are not selectively retained in a chamber or column to be flushed out of the chamber or column during the separation process. Usually, linear flow is indicated in cm/hour (h). Volumetric flow rates are indicated in ml/h or ml/minute (min). The conversion between linear and volumetric flow rate can be carried out by those skilled in the art by using formulae known in the art.

The liquid used may be any liquid that allows the cells to remain viable in the medium during the period of time during which the method of enriching and/or isolating a target cell population is being carried out. In some embodiments the liquid is an aqueous medium. The liquid may for instance be the same medium as the medium in which the cells are suspended when they are being introduced into the separation compartment. The liquid will generally be selected according the specific requirements of the cells to which the method is applied, in particular the cells to be enriched, purified and/or isolated. As two illustrative examples, the liquid may be an oxygenated liquid and/or a nutrient-containing liquid. The liquid may for instance include a predetermined amount of a gas such as oxygen, nitrogen or carbon dioxide, of a compound, such as a peptide, a protein, a nucleotide, an oligonucleotide, a nucleic acid, a lipid, a saccharid, a vitamin, an ion, or a low molecular organic or inorganic compound. The liquid may for instance include a predetermined amount of a factor selected from the group consisting of a growth factor, a differentiation factor, a metabolite, a hormone, a drug, a drug candidate, a prodrug, a vitamin, and an antibiotic. The fluid may also be of a predetermined property such as a selected oxygen tension, carbon dioxide content, a selected temperature or a selected shear flow. The liquid may include a chelating agent such as EDTA, e.g. 1 mM thereof. The liquid may also include a buffer compound. Numerous buffer compounds are used in the art and may be used to carry out the various process steps described herein. Examples of buffers include, but are not limited to, solutions of salts of phosphate, carbonate, succinate, citrate, acetate, formate, barbiturate, oxalate, lactate, phthalate, maleate, cacodylate, borate, N-(2-acetamido)-2-amino-ethanesulfonate (also called (ACES), N-(2-hydroxyethyl)-piperazine-N'-2-ethanesulfonic acid (also called HEPES), 4-(2-hydroxyethyl)-1-piperazine-propanesulfonic acid (also called HEPPS), piperazine-1,4-bis(2-ethanesulfonic acid) (also called PIPES), (2-[Tris-(hydroxymethyl)-methylamino]-1-ethansulfonic acid (also called TES), 2-cyclohexylamino-ethansulfonic acid (also called CHES) and N-(2-acetamido)-iminodiacetate (also called ADA). Any counter ion may be used in these salts; ammonium, sodium, and potassium may serve as illustrative examples. Further examples of buffers include, but are not limited to, triethanolamine, diethanolamine, ethylamine, triethylamine, glycine, glycylglycine, histidine, tris(hydroxymethyl)aminomethane (also called TRIS), bis-(2-hydroxyethyl)-imino-tris(hydroxymethyl)methane (also called BIS-TRIS), and N-[Tris(hydroxymethyl)-methyl]-glycine (also called TRICINE), to name a few. Two illustrative examples of ionic liquids are 1,3-dialkylimidazolium-tetrafluoroborates and 1,3-dialkylimidazolium-hexafluoroborates. The ionic liquid may be a polar ionic liquid such as for instance 1-ethyl-3-methylimidazolium tetrafluoroborate, N-butyl-4-methylpyridinium tetrafluoroborate, 1,3-dialkylimidazolium-tetrafluoroborate, 1,3-dialkylimidazolium-hexafluoroborate, 1-ethyl-3-methylimidazolium bis(pentafluoroethyl)-phosphinate, 1-butyl-3-methylimidazolium tetrakis(3,5-bis(trifluoromethylphenyl)borate, tetrabutyl-ammonium bis(trifluoromethyl)imide, ethyl-3-methylimidazolium trifluoromethanesulfonate, 1-butyl-3-methylimidazolium methylsulfate, 1-n-butyl-3-methylimidazolium ([bmim]) octylsulfate, and 1-n-butyl-3-methylimidazolium tetrafluoroborate. The ionic liquid may be a non-polar ionic liquid such as for instance, 1-ethyl-3-methylimidazolium bis[(trifluoromethyl)-sulfonyl]amide bis(triflyl)amide, 1-ethyl-3-methylimidazolium bis[(trifluoromethyl)sulfonyl]-amide trifluoroacetate, 1-butyl-3-methylimidazolium hexafluorophosphate, 1-hexyl-3-methylimidazolium bis(trifluoromethylsulfonyl)imide, 1-butyl-3-methylimidazolium bis(trifluoromethylsulfonyl)imide, trihexyl(tetradecyl)phosphonium bis[oxalato(2-)]borate, 1-hexyl-3-methyl imidazolium tris(pentafluoroethyl)trifluorophosphate, 1-butyl-3-methyl-imi-dazolium hexafluorophosphate, tris(pentafluoroethyl)trifluorophosphate, trihexyl(tetradecyl)-phosphonium, N"-ethyl-N,N,N',N'-tetramethylguanidinium, 1-butyl-1-methyl pyrroledinium tris(pentafluoroethyl) trifluorophosphate, 1-butyl-1-methyl pyrrolidinium bis(trifluoromethylsulfonyl) imide, 1-butyl-3-methyl imidazolium hexafluorophosphate, 1-ethyl-3-methyl-imidazolium bis(trifluoromethylsulfonyl)imide and 1-n-butyl-3-methylimidazolium. In some embodiments the fluid is a culture medium. In some embodiments the liquid includes phosphate buffered saline.

The plurality of cells is exposed to a permanent flow of liquid. Typically the cells are constantly held in suspense, such that the cells are not allowed to settle. The porous medium that is included in the outlet prevents the cell population from exiting the separation compartment.

A flow of liquid may in some embodiments be achieved by using a pump. A variety of suitable pump systems, typically on the basis of a pressure pump, are used in the art. In some embodiments manual force may be applied, for example using a syringe. The flow rate will be selected according to the volume of the separation compartment and the type of cells to be isolated. In typical embodiments the flow rate is selected to be high enough to prevent the cells from settling within the separation compartment. Furthermore, the flow rate is generally selected to be low enough to prevent any injury/damage to the cells.

A continuous flow of fluid serves in suspending target cells. A continuous flow can be adjusted to a flow rate where the target cell(s) is/are maintained in a position that can be envisaged as a floating composition. In such a position the target cell(s) is/are exposed to a gravitational force, which typically creates a force in the direction of the ground. This direction may correspond to the direction of the base of the separation compartment. A continuous flow of a liquid acts to create a force which opposes the gravitational force, and the resultant vector summation of all forces acting on the cells defines an equilibrium state where each cell is independently suspended without the need for support. In some embodiments the forces substantially immobilize the cells by the summation of the vector forces acting on the cells. In some embodiments the forces cause turbulence, which keeps the cell(s) in flow. Determining factors with regard to the forces acting on the cell(s) are inter alia the density, mass and shape of the cell(s). As an illustrative example, the forces of the flow will generally be weaker in comparison to gravity for cells of higher mass, so that cells with a higher mass will usually be floating to a lower extent, for instance at a lower level, than cells with a lower mass. Therefore cells can be separated according to their size and density. Channeling, turbulence and backmixing may usually be allowed to occur in a method as described herein.

In some embodiments the continuous flow of fluid can be taken to be a continuous perfusion of media. Under a continuous flow of fluid the cell(s) is/are in an environment of minimal shear and pressure, and one which provides a constant supply of oxygen and nutrients to the cell(s). Generally the cell(s) thus remain(s) viable. Cells isolated, enriched and/or purified according to a method as described herein can therefore usually be used for any desired application. In particular in embodiments where the position of the outlet in the separation compartment is arranged in such a way that the continuous flow of liquid prevents the target cell or the target cell population from exiting the separation compartment via the outlet, the target cell(s) may be kept at a certain position in the separation compartment due to an equilibrium between forces of gravitation and flow (supra). In embodiments where the separation compartment is designed in such a way that the porous medium prevents the cell population from exiting the separation compartment via the outlet.

The position of a known target cell and its separation from undesired cells may be affected by adjusting the liquid that is used under an essentially constant flow. As long as the target cell remains viable, conditions such as viscosity, density, the flow of liquid, and the inclination of the separation compartment - such as the inclination of an axis thereof relative to the force of gravity - may for instance be adjusted. Further suitable factors are within the skilled person's knowledge.

Applying an at least essentially linear flow of liquid, e.g. wash buffer, via the inlet of the separation compartment may allow the plurality of cells to flow through the separation compartment, for instance through a filtration column or filtration chamber and the porous medium. In some embodiments of a method disclosed herein a negative or depletion approach for isolating target cells from a plurality of cells in a fluid sample is used. For example, cells having a maximal width that is lower than the pore size of a porous medium in the microfiltration unit are completely washed through the porous medium, and thus exit the separation compartment via the outlet. They are thereby efficiently removed from the plurality of cells. In contrast, the removal of target cells that have a maximal width that is larger than the pore size of the porous medium from the separation compartment, e.g. filtration column or chamber, is prevented. In embodiments where an at least essentially linear flow is used, it typically serves as an upward force (or buoyancy) for the cells which can be kept at a specific spatial position within the separation column or chamber, dependent on their cell density. As indicated above, the applied flow of wash buffer is typically an at least essentially linear flow.

The distance between inlet and outlet may be selected as desired. Likewise any volume of the separation compartment way be used, as long as the volume is at least several thousand-fold the expected volume of the target cell, or of the target cell population, i.e. the total volume of all cells of the target cell population. Typically the volume of the separation compartment will 1 cm³ or more, such as 10 cm³ or more. When using separation compartments with a longitudinal axis, in particular with an essentially uniform maximal width along the longitudinal axis, the inventors have observed that typically separation increases with the length of the longitudinal axis.

A method as described herein may in some embodiments be used with a predefined plurality of cells, which is then introduced into the separation compartment, whereafter no further cells are being introduced therein. In some embodiments a continuous supply of cells may continuously be introduced into the separation compartment, while a continuous flow of a suitable liquid is being applied. Undesired cells may continuously be washed out of the separation compartment, while desired target cells remain in the separation compartment. As an illustrative example, cells may be removed from a stationary bioreactor and transferred to a separation compartment while the media is transmitted to and through the separation compartment. In some embodiments with a continuous supply of cells a continuous enrichment of target cells may be performed.

In some embodiments the separation compartment may be agitated for any desired period of time while an at least essentially constant flow of liquid is allowed to pass through the separation compartment. A shaker may for instance be used to agitate the separation compartment. The present inventors have further found that it is generally beneficial to support a filtration process of a method according to the invention by agitating the separation compartment by a shaker, e.g., a laboratory shaker. In some embodiments a fast rotating step (step 200 to 250) is used. This measure typically helps to better distribute the target cells in the separation compartment. Where desired the outflowing liquid exiting the separation compartment may be collected. In one embodiment eluate fractions may be collected, for example to monitor the elution of cells.

In some embodiments of a method of enriching, isolating and/or purifying a target cell population the inlet is closed after an at least essentially constant flow of liquid has been allowed to pass through the separation compartment. If desired, the separation compartment maybe shaken briefly, for example in order to resolve certain cells such as erythrocytes settled down on the porous medium. In some embodiments where the separation compartment is not arranged in an orientation where the outlet is positioned to face at least essentially in the direction of gravity, the separation compartment is brought into such an orientation. The outlet is opened if it is in a closed state. For this purpose, a pump can be used.

After applying an essentially constant flow of a suitable liquid to pass through the separation compartment a fluid is in some embodiments allowed to enter the separation compartment via an inlet. This fluid is at least essentially immiscible with the liquid currently encompassing the target cell population within the separation compartment. Furthermore the fluid has a density that is lower than the density of the fluid currently encompassing the target cell population. In some embodiments the fluid is a gas which may include nitrogen, oxygen and/or carbon monoxide. In some embodiments the respective gas is air. Liquid, which encompasses the target cell population, is allowed to exit via the outlet. In some embodiments a portion of the liquid is allowed to exit the separation compartment. In some embodiments the entire liquid is allowed to exit the separation compartment. Typically the liquid level of the liquid currently encompassing the target cells is thereby lowered to reduce the volume of the cell suspension that is included in the separation compartment. In some embodiments the liquid level is lowered to the surface of the membrane filter by the introduction of air to the inlet, which may for example be located at the upper end of the microfiltration unit. As a result the volume of the liquid that encompasses the target cell population is allowed to decrease.

Subsequently, the porous medium may be removed from the outlet. The porous medium can be used, e.g., for microscopic analysis, for storage or for further isolation of DNA. Storage can be carried out at any desired condition known to be suitable for the intended purpose. As an illustrative example, storage at -20 °C in a sufficient amount of a buffer solution such as 200 microliter PBS may be used. The membrane filter can also be used for storage or for further isolation of the DNA, RNA or proteins of the isolated target cells.

Any step of a method as described in this specification, such as the use of the separation compartment, including introducing a plurality of cells or a fluid into the separation, arranging the separation compartment into a desired orientation may be carried out manually, automatically or in a combination thereof. An automatic use may include a control system that induces in a programmed manner the performance of any acts desired for a process to be carried out using the device of the invention. Examples include, but are not limited to, opening or closing an inlet or an outlet, operating a pump, injecting, stirring, the application of energy and the start of a detection device. The use may be an integrated step of a method described herein. Such a method may include a number of sequential steps. An illustrative initial step of a method of processing a plurality of cells as described herein may be aimed at a volume reduction and include for example centrifugation. A late step of a method of enriching, isolating and/or purifying a cell from a respective plurality of cells may include the detection of desired cells, for instance by means of an automated cell counting device.

As already indicated above, the separation compartment is in some embodiments for instance an external, internal or integrated part of a larger apparatus. Such an apparatus may for instance include means such as screws and holders to alter and to fix the position of the separation compartment. Further illustrative elements of a respective apparatus include means for temperature regulation such as a cooling device, a heating device and a temperature controller. In some embodiments the apparatus includes further components adapted to perform any function desired to be performed. An illustrative example of a component that may be part of an inlet or an outlet or fluidly coupled to an inlet or an outlet is a valve. Yet a further illustrative example of a component that may be part of a respective apparatus is a spectrometer such as a fluorescence spectrometer, which may serve in detecting outflow or inflow of the separation compartment, including fractions eluting from the separation compartment.

Two embodiments of carrying out a method of enriching, purifying and/or isolating one or more target cells as described herein are illustrated in Fig. 1. As shown in the following Examples, tumour cells could be isolated from human blood with high recovery rate, in a model system. Furthermore, the isolated tumour cells contained a high portion of optically intact cells, as verified by microscopy, indicating that this method not only allows high re-identification rates of target cells from fluid samples but also gentle recovery of viable target cells. In some embodiments the plurality of cells may be human blood and the target cell population may be leukocytes, including a population of lymphocytes. In some embodiments the plurality of cells may be human blood and the target cell population may be cord blood cells.

Disclosed herein are methods for enriching, purifying and/or isolating target cells, typically from a fluid sample such as a liquid sample. A respective method may be taken to combine several separation methods within a single process, including a single working step. An example of a population of target cells are circulating tumour cells such as tumour cells of epithelial origin. Such tumour cells differ from most of the normal cellular components in blood of a healthy person in their size and density. As shown in the following Examples, using an at least essentially linear flow or a turbulent/counter-flow of buffer solution, e.g., a wash buffer, the outflow of tumour cells from the separation compartment, e.g., a separation or filtration column is prevented by a porous membrane with a specific pore size, of, e.g., 5 micrometres, which is chosen smaller than the size of the tumour cells. Blood cells with lower size (and higher density) are generally completely washed through the porous membrane filter. In addition, the linear or turbulent flow of buffer serves as an upward force for cells which are kept at a specific spatial position within the separation compartment, depending on their cell density. The density of tumour cells is lower than that of blood cells. The distribution of the target cells within the liquid used can be further assisted by shaking the microfiltration unit during the filtration process. Accordingly, a method as described herein can be taken to combine cell size- and cell density-dependent separation methods for the isolation, purification and/or enrichment of target cells in fluid samples such as circulating tumour cells in blood. An at least essentially linear or turbulent fluid flow in a respective method of also prevents the target cells from getting into contact with the porous medium, e.g. a porous membrane, thereby preventing a potential mechanical damage of the cells. As a result a corresponding method allows a particularly gentle isolation, purification and/or enrichment of living and viable target cells. After successful removal of undesired blood components, the liquid level in the separation column is lowered so that the concentration of target cells is increased. Where desired, the liquid level may be lowered to such an extent that the remaining cells are collected on the porous medium from where they can be isolated. In this way, a very efficient isolation and enrichment of circulating tumour cells in blood could be achieved, as demonstrated in the following Examples.

Briefly, in the Examples a specific number of pancreatic tumour cells has been added to blood of a healthy person, in a model system. The number of introduced tumour cells has been chosen to be too low for a direct detection of the tumour cells by currently used extremely sensitive and specific techniques such as PCR. In order to allow visual quantification of the tumour cells on the porous membrane, isolated by the methods of the invention, vital staining was used on the tumour cells. Subsequently, DNA was isolated from the stained cells and detected by PCR. Both a visual test and PCR demonstrated successful enrichment of the tumour cells from the blood sample by the method described herein. In comparison to an assay for direct detection of the tumour cells in the blood sample using, an at least 200 fold reduction of the detection level could be achieved, using a method as defined above. Accordingly, a method as disclosed in this specification is typically characterized advantageously by an improved recovery and enrichment rate. Furthermore, generally no pre-treatment of a blood sample is required for carrying out a method as disclosed herein. In addition, generally no intervention is necessary during a separation progress that falls under a method of enriching, isolating and/or purifying a cell from a respective plurality of cells as described herein. The cells can usually be isolated in a vital stage and can be used not only for subsequent analytical methods, for example, for the detection of mutations or other pathologically relevant amendments, but also for tissue culture, for instance, in order to establish a primary tumour cell line.

In light of this, the methods of the present invention allow for the isolation, purification and/or enrichment of one or more cells from fluid samples such as blood, which deviate from normal cellular components of blood in size and/or density. Examples of such cells are foetal cells in maternal blood or cells which migrate into blood vessels during the progression of a disease or which are being pathologically amended in blood. In this regard migrating or circulating tumour cells are often of particular interest. Such cells are also detectable in low concentration in blood of patients suffering from solid tumours. The isolation and/or enrichment of tumour cells from blood can be used for first diagnosis of a tumorous disease but also for monitoring of recurrence of the tumorous disease and/or metastasis or tumour therapies. As evident to those skilled in the art, monitoring is of utmost economic significance, particularly in light of the tense financial situation in health care at present.

Circulating tumour cells (CTCs) likely derive from clones in the primary tumour, suggesting that they can be used for all biological tests applying to the primary cells. A method as described in this specification is typically a low-cost innovative technique, which can be taken to be a combination of different separation approaches, for example to isolate and sort tumour cells by size and/or density. A respective method is generally able to isolate rare, fixed, tumour cells, with a high recovery rate. Such cells are well preserved morphologically during a method described herein, due to the gentle isolation procedure used. The methods of the invention allow isolation of living cells able to grow in culture. High quality genetic materials can for instance be obtained directly from tumour cells isolated on a porous medium. Due to their versatility and capacity to isolate CTCs within short time, a method described herein is for example also able to simplify and improve non-invasive access to tumour cells.

In some embodiments a methods as described in this document can be followed by quantitative and qualitative analysis of the, thus, enriched, purified and/or isolated target cells. Numerous quantitative and qualitative cytological assays are well known to those skilled in the art. For example, morphological analysis, including, e.g., nucleo-cytoplasmic ratio, nuclear details and size of nucleoli of the isolated cells can be carried out by microscopy. Furthermore, the enriched and/or isolated target cells can be used for the establishment of primary tissue cultures. Alive isolated circulating tumour cells (CTCs) can be used for testing their potential capacity to initiate tumour formation in animal models and are easily accessible to a large range of molecular biological analysis. In addition, enumeration and immune-cytochemical methods can be performed for identifying the tumoral origin of circulating tumour cells. Furthermore, the cells obtained using a method described herein can also be used for isolation and/or analysis of the cellular DNA, RNA or proteins. For example, the isolated DNA can be used to detect tumour specific mutations, for example by means of PCR.

Accordingly, in some embodiments a method of isolating, purifying and/or enriching a target cell or a population of target cells, includes quantitative and/or qualitative analysis of the enriched, purified and/or isolated target cells, for instance mutation analysis.

In some embodiment of a respective method of isolating, purifying and/or enriching a target cell or a population of target cells, the recovery rate of the target cell is at least 10.000 cells, at least 7.500 cells, or even at least 5000 cells per ml fluid sample.

For the direct detection of specific mutations in tumour cells circulating in the blood by PCR about 1 million cells per ml blood are currently required. As shown in the following Examples, the detection limit for specific mutations could be reduced to less than 5.000 cells per ml blood by the methods of the invention. This corresponds to an at least 200 fold enrichment, in comparison to the methods described in the art, in particular when using microfiltration in combination with turbulent flow. In some embodiments the enrichment of the target cell is at least 50 fold, at least 75 fold, at least 100 fold, at least 150 fold or even at least 200 fold.

In some embodiments of a method as described above, an automated system is used.

In some embodiments the automated system is a device that includes at one or more separation compartments as described above, e.g. a filtration chamber, automated means for providing fluid flow through the microfiltration unit, and a power source for providing fluid flow. In some embodiments a respective automated system further includes means for providing a signal source for the generation of forces on active chips. An automated system used in a method as described above can also include one or more active chips, separation chambers, or separation columns.

The contents of the articles, patents, and patent applications, and all other documents and electronically available information mentioned or cited herein, are hereby incorporated by reference in their entirety to the same extent as if each individual publication was specifically and individually indicated to be incorporated by reference. In case of conflict, the present specification, including definitions, will control. Applicants reserve the right to physically incorporate into this application any and all materials and information from any such articles, patents, patent applications, or other physical and electronic documents.

The listing or discussion of a previously published document in this specification should not necessarily be taken as an acknowledgement that the document is part of the state of the art or is common general knowledge.

The methods, uses and combinations illustratively described herein may suitably be practiced and applied in the absence of any element or elements, limitation or limitations, not specifically disclosed herein. Additionally, the terms and expressions employed herein have been used as terms of description and not of limitation, and there is no intention in the use of such terms and expressions of excluding any equivalents of the features shown and described or portions thereof. It is recognized that various modifications are possible within the scope of the invention claimed. Thus, it should be understood that although the present invention has been specifically disclosed by exemplary embodiments and optional features, modification and variation of the inventions embodied therein herein disclosed may be resorted to by those skilled in the art, and that such modifications and variations are considered to be within the scope of this invention.

The invention has been described broadly and generically herein. Each of the narrower species and subgeneric groupings falling within the generic disclosure also form part of the methods, uses and combinations. This includes the generic description of the methods, uses and combinations with a proviso or negative limitation removing any subject matter from the genus, regardless of whether or not the excised material is specifically recited herein.

Other embodiments are set forth within the appending claims. In addition, where features or aspects of the invention are described in terms of Markush groups, those skilled in the art will recognize that the invention is also thereby described in terms of any individual member or subgroup of members of the Markush group.

The invention is further illustrated by the following non limiting examples and the appended figures. As one of ordinary skill in the art will readily appreciate from the present disclosure, other compositions of matter, means, uses, methods, or steps, presently existing or later to be developed that perform substantially the same function or achieve substantially the same result as the corresponding exemplary embodiments described herein may likewise be utilized according to the present invention.

### EXEMPLARY EMBODIMENTS OF A METHOD OF THE INVENTION

**Figure 1A** shows the setup and implementation of the method of the invention using microfiltration in combination with linear flow for enriching and/or isolating target cells from a fluid sample is shown. The microfiltration unit comprises an inflow at the upper end, an outflow at the lower end, and a membrane filter at the lower end. In this embodiment of the method of the invention, the microfiltration unit is turned around after application of the fluid sample, so that the filter membrane and the outflow are located at the upper end of the microfiltration unit, whereas the inflow of the wash buffer occurs via the inflow localized at the opposite lower end. The microfiltration unit is either kept in an upright position or can be inclined at a certain angle. The filtration process can be supported by agitating the microfiltration using a shaker. Thereafter, the microfiltration is turned again so that now the membrane filter and the outflow are located at the lower end whereas the inflow is at the upper end. Further wash buffer is added to the inflow at the upper end. Finally, the fluid level is lowered by the application of air. The filter membrane is removed from the microfiltration unit for storage or for further quantitative or qualitative analysis of the isolated and/or enriched target cells.

**Figure 1** **B** shows the setup and implementation of a method of the invention using counter-flow microfiltration, in combination with turbulent flow is shown. First, a fluid sample such as a blood sample is applied to a counter-flow microfiltration unit. This microfiltration unit comprises a port which allows for ventilation of the microfiltration unit, an inflow and an outflow and a membrane filter. The port is localized at one end of the microfiltration unit and the inflow, outflow and membrane filter are at the opposite end of the microfiltration unit. Further, the membrane filter is upstream of the outflow and near the inflow in order to allow a counter-flow mode. Then, a turbulent flow of wash buffer is applied to the inflow of the microfiltration unit, by using a pump. The filtration process is supported by shaking the microfiltration unit by a shaker. Eluate fractions can be collected to monitor the process. Thereafter, the inflow is closed and the fluid level in the microfiltration unit is lowered by the admission of air to the port by using a pump in order to allow the removal of the membrane filter. The filter membrane can be used for further quantitative or qualitative analysis of the isolated and/or enriched target cells or stored.

**Figure 5** shows an example of assembling a filtration column in downward orientation and upward orientation. For a Downward orientation of the outlet of the separation compartment an Original-Perfusor-Syringe OPS 20ml Luer Lock Duo 2.0x30mm (B.Braun Melsungen AG, 34209 Melsungen, Germany, **1**) is being equipped with a 13mm Swinnex Syringe Filter Holder with a hydrophilic Polycarbonate Membrane Filter of 5µm pore size, 13mm (Sterlitech Cooperation, 22027 70th Avenue S, Kent, WA 98032-1911 USA, **2**) and an Original-Perfusor hollow needle 2.0x30mm (**3**). The needle is part of Original-Perfusor-Syringe (supra). Furthermore, a Venofix Luer Lock 0.8x20mm (B.Braun Melsungen AG, 34209 Melsungen, Germany, **4**), a Discofix C stopcock (B.Braun Melsungen AG, 34209 Melsungen, Germany, **5a**), a 360° stopcock, 4-way, blue (pvb medizintechnik gmbh & co kg, Hauptstraße 45-47, 85614 Kirchseeon, Germany, **5b**), and flexible tubes and connecting pieces (Bio-Rad Laboratories Headquarters, 1000 Alfred Nobel Drive, Hercules, CA 94547 USA, **6**) are being mounted to the syringe. For an Upward orientation of the outlet of the separation compartment an Original-Perfusor-Syringe OPS 20ml Luer Lock Duo 2.0x30mm (supra, **1**) is being equipped with a 13mm Swinnex Syringe Filter Holder with a hydrophilic Polycarbonate Membrane Filter of 5µm pore size, 13mm (supra, **2**), an Original-Perfusor hollow needle 2.0x30mm (**3**), and flexible tubes and connecting pieces (Bio-Rad Laboratories supra, **4**) are being assembled.

Presented are two experimental setups that have been used to show the proof-of-principle of the methods of the present invention which combine a cell-size dependent separation method, i.e. microfiltration with a cell-density dependent separation method, by using linear flow or turbulent flow/counter-flow. The linear flow arrangement is shown in Fig. 1A, whereas the turbulent flow setup is depicted in Fig. 1B. Successful enrichment of cellular subpopulations from human blood could be achieved already in the linear flow setup.

In order to enrich and/or isolate tumour cells from a human blood sample by a microfiltration process utilizing a linear flow as shown in Figure 1, the following steps have been carried out:
Step 1: Labelling of the cells for subsequent microscopic identification. As source material, pancreatic tumour cells PaTu 8988S with an average cell diameter of 11.11 plus/minus 0.07 micrometre and PaTu 8988T with an average cell diameter of 7.17 plus/minus 0.05 micrometer, respectively, have been used. The pancreatic tumour cells which grow as adherent cells have been labelled with CytoTrackerGreen: CMFDA (5-chloromethylfluorescindiacetat)-Green (Invitrogen / Mol. Probes), 5 µM in serum-free medium by incubation at 37° C / 5% CO₂ for 30-45 minutes in order to allow re-identification of the cells.
Step 2: Removal of the adherent pancreatic tumour cells from the cell culture dish by incubation with the protease trypsin, centrifugation and re-suspension in full medium, determination of the number of cells.
Step 3: Introduction of a certain number of pancreatic tumour cells into a blood sample. 5 ml blood diluted with 5 ml PBS⁻⁻/EDTA dilution (1 mM EDTA, PBS⁻⁻: phosphate buffered saline without divalent cations). The blood used was fresh EDTA whole blood, without any additional treatment. 25.000 pancreatic tumour cells have been transferred to 10 ml of the diluted blood.
Step 4: Transfer of the blood suspension to a filtration column (cf. above).
Step 5: Microfiltration has been carried out as follows:
i): angle of inclination of filtration column: 0 °, upright (vertical), with membrane filter at the upper end, shaker: level 200-250 (fast), flow rate: 200 ml/h, flow volume: 200 ml, used wash buffer: PBS⁻⁻/1 mM EDTA.
ii): 180° rotation of the column (filter membrane at the lower end), the column has been shaken gently in order to resolve erythrocytes precipitated on the filter membrane, filtration, flow rate: 200 ml/h, flow volume: 100 ml; used wash buffer: PBS⁻⁻/1 mM EDTA; subsequently, lowering of the liquid level to the surface of the filter membrane by introducing air.
Step 6: Removal of the membrane filter and microscopic analysis or storage in 200 µl PBS⁻⁻ at -20°C, for example, for subsequent reprocessing or analysis / isolation of the DNA of the tumour cells.

In order to enrich and/or isolate tumour cells from a human blood sample by a microfiltration process utilizing a turbulent flow as shown in Figure 2, the following steps have been carried out:
Steps 1 to 3 correspond to steps 1 to 3, respectively, indicated in section 1.1.
Step 4: Transfer of the blood suspension to the filtration column. For this purpose, the membrane filter has been removed temporarily; setting of the filtration column into an upright position, membrane filter at the lower end, port below the filter membrane closed, to prevent leakage of liquid through the membrane (port not shown in Figure 2), filling the column with PBS⁻⁻/EDTA (1 mM) with open port for ventilation.
Step 5: Microfiltration: Inclination angle of column: 0°, upright column, filter membrane at the lower end; shaker: Level 200-250 (fast), flow rate: 200 ml/h, flow volume: 200 ml, wash buffer: PBS⁻⁻/ EDTA (1 mM), brief and gentle shaking of the column in order to resolve erythrocytes precipitated on the filter membrane, subsequently lowering of the liquid level to the surface of the filter membrane by introducing air via the port.
Step 6: Removal of the membrane filter and microscopic analysis or storage in 200µl PBS⁻⁻ at -20°C for subsequent reprocessing or analysis / isolation of the DNA of the tumour cells.

## Claims

1. A method of enriching and/or isolating a target cell population from a plurality of cells in the presence of a gravitational force, the method comprising:
(a) introducing the plurality of cells into a separation compartment,
wherein the separation compartment has an inlet and an outlet fluidly coupled to the ambience, the separation compartment being defined by a circumferential wall, a base and a top,
the base and the top being arranged in an at least essentially opposing relationship within the separation compartment;
(b) in an arrangement of the separation compartment where the outlet is positioned to face within an angle of ± 75° opposite to the direction of the gravitational force, introducing an at least essentially constant flow of a suitable liquid through the inlet of the separation compartment, and allowing an at least essentially constant flow of liquid to exit the separation compartment via the outlet, thereby suspending the plurality of cells in a continuous flow of liquid, such that the continuous flow of liquid prevents the target cell population from sedimenting and from exiting the separation compartment via the outlet;
thereby enriching and/or isolating the target cell population.

2. The method of claim 1, wherein the outlet comprises a porous medium having pores of a size smaller than the average cell size of cells of the target cell population, the method further comprising:
(c) introducing a fluid into the separation compartment through the inlet thereof, wherein the fluid is at least essentially immiscible with the liquid encompassing the target cell population in the separation compartment and of a density that is lower than the same, allowing liquid encompassing the target cell population to exit via the outlet, such that the volume of the same is allowed to decrease.

3. The method of any one of the preceding claims, wherein:
(a) introducing an at least essentially constant flow of a suitable liquid through the inlet of the separation compartment is carried out after arranging the separation compartment in such an orientation that the outlet is positioned to face at least essentially opposite to the direction of the gravitational force, and/or
(b) the outlet is comprised in the base or in the top, and/or
(c) the inlet and the outlet are arranged in an at least essentially opposing relationship with each other.

4. A method of enriching and/or isolating a target cell population from a plurality of cells, the method comprising:
(a) introducing the plurality of cells into a separation compartment,
wherein the separation compartment has an inlet and an outlet fluidly coupled to the ambience, the separation compartment being defined by a circumferential wall, a base and a top,
the base and the top being arranged in an at least essentially opposing relationship within the separation compartment, wherein the base comprises the outlet, and wherein the outlet comprises a porous medium having pores of a size smaller than the average cell size of cells of the target cell population;
(b) introducing an at least essentially constant flow of a suitable liquid through the inlet of the separation compartment, and allowing an at least essentially constant flow of liquid to exit the separation compartment via the outlet, thereby suspending the plurality of cells in a continuous flow of liquid, such that the continuous flow of liquid prevents the target cell population from sedimenting, and the porous medium prevents the target cell population from exiting the separation compartment via the outlet;
thereby enriching and/or isolating the target cell population.

5. The method of claim 4, further comprising:
(c) introducing a fluid into the separation compartment through the inlet thereof, wherein the fluid is at least essentially immiscible with the liquid encompassing the target cell population in the separation compartment and of a density that is lower than the same, allowing liquid encompassing the target cell population to exit via the outlet, such that the volume of the same is allowed to decrease.

6. The method of claim 4 or 5, wherein
(a) the method is being carried out in the presence of a gravitational force, wherein introducing an at least essentially constant flow of a suitable liquid through the inlet of the separation compartment is carried out after arranging the separation compartment in such an orientation that the outlet is positioned to face within an angle of ± 75° in the direction of the gravitational force, after introducing the plurality of cells into the separation compartment, and/or
(b) the separation compartment is arranged in such an orientation that the outlet is positioned to face at least essentially in the direction of the gravitational force, while introducing the at least essentially constant flow of a suitable liquid through the inlet of the separation compartment.

7. The method of any one of the preceding claims, wherein:
(a) the method is being carried out in the presence of a gravitational force, wherein the at least essentially constant flow of liquid introduced through the inlet of the separation compartment is allowed to create a force on the target cell population that opposes the gravitational force, and/or
(b) the fluid introduced into the separation compartment is a gas, and/or wherein the fluid introduced into the separation compartment is air, and/or
(c) the method further comprises inclining the separation compartment relative to its original orientation, after introducing the plurality of cells into the separation compartment, and/or
(d) the enrichment of the target cell population is at least 50 fold, 75 fold, 100 fold, 150 fold or 200 fold, and/or
(e) the method further comprising agitating the separation compartment after the same has been arranged in an orientation where the outlet is positioned to face at least essentially in the direction of the gravitational force.

8. The method of any one of the preceding claims, wherein:
(a) the circumferential wall has a wall portion that has an at least essentially straight surface, and/or
(b) the circumferential wall has an at least essentially cylindrical shape, and/or
(c) the inlet is (i) arranged proximate to the outlet or (ii) comprised in the top, and/or
(d) the separation compartment has a longitudinal axis, wherein optionally the separation compartment has an at least essentially uniform maximal width along the longitudinal axis, and/or
(e) the circumferential wall defines an at least essentially tubular portion of the separation compartment, and/or
(f) the separation compartment is comprised in a housing, and/or
(g) the liquid introduced through the inlet of the separation compartment is an aqueous liquid.

9. The method of any one of the preceding claims, being carried out in the presence of a gravitational force, the method further comprising arranging the separation compartment in such an orientation that the outlet is positioned to face within an angle from + 75° to - 75° of the gravitational force, before the fluid, which is at least essentially immiscible with the liquid encompassing the target cell population, is introduced into the separation compartment,
wherein the separation compartment is optionally arranged in such an orientation that the outlet is positioned to face at least essentially in the direction of the gravitational force, before the fluid, which is at least essentially immiscible with the liquid encompassing the target cell population, is introduced into the separation compartment.

10. The method of any one of the preceding claims, wherein the separation compartment has a longitudinal axis, and wherein the outlet is arranged at least essentially at the longitudinal axis, wherein optionally arranging the separation compartment in such an orientation that the outlet is positioned to face at least essentially in the direction or opposite to the direction of the gravitational force comprises bringing the separation compartment into a position where the longitudinal axis is at least essentially parallel to the gravitational force.

11. The method of any one of the preceding claims, wherein the separation compartment comprises a single inlet, or wherein the separation compartment comprises two inlets.

12. The method of claim 11, wherein the separation compartment comprises two inlets and wherein one inlet is arranged proximate to the outlet and wherein introducing an at least essentially constant flow of a suitable liquid through the inlet of the separation compartment is carried out after arranging the separation compartment in such an orientation that the outlet is positioned to face at least essentially in the direction of the gravitational force.

13. The method of any one of the preceding claims,
(a) wherein the plurality of cells is comprised in at least one of a body fluid sample, an environmental sample, a cell culture sample, a bone marrow sample, a sewage sample, a food sample, a milk sample, a forensic sample, a biological molecule production sample, a protein preparation sample, a lipid preparation sample, a carbohydrate preparation sample, and any combination thereof,
wherein optionally the body fluid sample is one of a blood sample, a serum sample, an amniotic fluid sample, a semen sample, a lymphatic fluid sample, a cerebrospinal fluid sample, a nasopharyngeal wash sample, a sputum sample, a mouth swab sample, a throat swab sample, a nasal swab sample, a bronchoalveolar lavage sample, a bronchial secretion sample, and a urine sample; and/or
(b) wherein the target cell population is a population of somatic cells, a population of stem cells or a population of progenitor cells, and/or
wherein the target cell population is a population of one of tumour cells, foetal cells, and embryonic cells.

14. The method of any one of claims 1 to 3, and 6 to 13, wherein arranging the separation compartment in such an orientation that the outlet is positioned to face within an angle from + 75° to - 75° in the direction of the gravitational force or within an angle from + 75° to - 75° opposite to the direction of the gravitational force comprises rotating the separation compartment.

15. The method of any one of the preceding claims, further comprising:
(d) collecting the liquid encompassing the target cell population together with the target cell population from the separation compartment.

## Patentansprüche

1. Verfahren zum Anreichern und/oder Isolieren einer Zielzellpopulation aus einer Vielzahl von Zellen in Gegenwart einer Gravitationskraft, umfassend:
(a) Einleiten einer Vielzahl von Zellen in ein Trennkompartiment, wobei das Trennkompartiment über einen flüssig mit der Umgebung gekoppelten Einlass und Auslass verfügt, das Trennkompartiment durch eine umlaufende Wand, eine Basis und einen Deckel begrenzt wird, die Basis und der Deckel in einer zumindest im Wesentlichen gegenüberliegenden Beziehung im Innern des Trennkompartiments angeordnet sind;
(b) in eine Anordnung des Trennkompartiments, in der der Auslass in einem Winkel von ± 75° entgegengesetzt der Gravitationskraft ausgerichtet ist, Einleiten eines zumindest im Wesentlichen konstanten Flusses einer geeigneten Flüssigkeit durch den Einlass des Trennkompartiments, und Ermöglichen des Austritts eines zumindest im Wesentlichen konstanten Flüssigkeitsflusses durch den Auslass, dabei Suspendieren der Vielzahl von Zellen in einem kontinuierlichen Flüssigkeitsfluss, sodass der kontinuierliche Flüssigkeitsfluss die Zielzellpopulation von der Sedimentation und vom Verlassen des Trennkompartiments durch den Auslass abhält;
dadurch Anreichern und/oder Isolieren der Zielzellpopulation.

2. Verfahren nach Anspruch 1, wobei der Auslass ein poröses Medium mit Poren einer kleineren Größe als der durchschnittlichen Zellgröße der Zielzellpopulation umfasst, weiterhin umfassend:
(c) Einleiten einer Flüssigkeit in das Trennkompartiment durch den Einlass desselben, wobei die Flüssigkeit zumindest im Wesentlichen nicht mischbar mit der die Zielzellpopulation umgebenden Flüssigkeit im Trennkompartiment und von einer niedrigeren Dichte als derselben ist, Ermöglichen des Austritts der die Zielzellpopulation umgebenden Flüssigkeit durch den Auslass, sodass das Volumen derselben sich vermindern kann.

3. Verfahren nach einem der vorherigen Ansprüche, wobei:
(a) das Einleiten eines im Wesentlichen konstanten Flusses einer geeigneten Flüssigkeit durch den Einlass des Trennkompartiments nach dem Anordnen des Trennkompartiments in solch einer Orientierung, dass der Auslass zumindest im Wesentlichen in der der Gravitationskraft entgegengesetzten Richtung ausgerichtet ist, ausgeführt wird, und/oder
(b) der Auslass in der Basis oder dem Deckel enthalten ist, und/oder
(c) der Einlass und der Auslass in einer im Wesentlichen gegenüberliegenden Beziehung zueinander angeordnet sind.

4. Verfahren zum Anreichern und/oder Isolieren einer Zielzellpopulation aus einer Vielzahl von Zellen, umfassend:
(a) Einleiten der Vielzahl von Zellen in ein Trennkompartiment, wobei das Trennkompartiment über einen mit der Umgebung flüssig gekoppelten Einlass und Auslass verfügt, das Trennkompartiment durch eine umlaufende Wand, eine Basis und einen Deckel begrenzt wird, die Basis und der Deckel in einer zumindest im Wesentlichen gegenüberliegenden Beziehung im Innern des Trennkompartiments angeordnet sind, wobei die Basis den Auslass umfasst und wobei der Auslass ein poröses Medium mit Poren einer kleineren Größe als die durchschnittliche Zellgröße der Zielzellpopulation umfasst;
(b) Einleiten eines im Wesentlichen konstanten Flusses einer geeigneten Flüssigkeit durch den Einlass in das Trennkompartiment, und Ermöglichen des Austritts eines zumindest im Wesentlichen konstanten Flüssigkeitsflusses aus dem Trennkompartiment durch den Auslass, dabei Suspendieren der Vielzahl der Zellen in einem kontinuierlichem Flüssigkeitsfluss, sodass der kontinuierliche Flüssigkeitsfluss die Zielzellpopulation vom Sedimentieren abhält, und das poröse Medium die Zielzellpopulation vom Austritt aus dem Trennkompartiment durch den Auslass abhält;
dadurch Anreichern und/oder Isolieren der Zielzellpopulation.

5. Verfahren nach Anspruch 4, ferner umfassend:
(c) Einleiten einer Flüssigkeit in das Trennkompartiment durch den Einlass desselben, wobei die Flüssigkeit zumindest im Wesentlichen nicht mischbar mit der die Zielzellpopulation im Trennkompartiment umgebenden Flüssigkeit und von einer geringeren Dichte als derselben ist, Ermöglichen des Austritts der die Zielzellpopulation umgebenden Flüssigkeit durch den Auslass, sodass das Volumen derselben sich vermindern kann.

6. Verfahren nach einem der Ansprüche 4 oder 5, wobei:
(a) das Verfahren in Anwesenheit einer Gravitationskraft ausgeführt wird, wobei das Einleiten eines zumindest im Wesentlichen konstanten Flusses einer geeigneten Flüssigkeit durch den Einlass des Trennkompartiments nach der Anordnung des Trennkompartiments in solch einer Orientierung, dass der Auslass in einem Winkel von ± 75° in Richtung der Gravitationskraft ausgerichtet ist, durchgeführt wird, nach Einleiten der Vielzahl von Zellen in das Trennkompartiment, und/oder,
(b) das Trennkompartiment in solch einer Orientierung angeordnet ist, dass der Auslass zumindest im Wesentlichen in Richtung der Gravitationskraft zeigt, während des Einleitens eines zumindest im Wesentlichen konstanten Flusses einer geeigneten Flüssigkeit durch den Einlass des Trennkompartiments.

7. Verfahren nach einem der vorherigen Ansprüche, wobei:
(a) das Verfahren in Anwesenheit einer Gravitationskraft ausgeführt wird, wobei der zumindest im Wesentlichen konstante Fluss einer durch den Einlass des Trennkompartiments eingeleiteten Flüssigkeit es ermöglicht, eine Kraft auf die Zielzellpopulation auszuüben, die der Gravitationskraft entgegenwirkt, und/oder
(b) das in das Trennkompartiment eingeleitete Fluid ein Gas ist, und/oder wobei das in das Trennkompartiment eingeleitete Fluid Luft ist, und/oder
(c) das Verfahren weiterhin Neigen des Trennkompartiments relativ zu seiner ursprünglichen Orientierung nach Einleiten der Vielzahl von Zellen in das Trennkompartiment umfasst, und/oder
(d) Anreichern der Zielzellpopulation mindestens 50-fach, 75-fach, 100-fach, 150-fach oder 200-fach ist, und/oder
(e) das Verfahren weiterhin Schütteln des Trennkompartiments umfasst, nachdem dasselbe in einer solchen Ausrichtung angeordnet wurde, dass der Auslass zumindest im Wesentlichen so positioniert ist, dass er zumindest im Wesentlichen in Richtung der Gravitationskraft zeigt.

8. Verfahren nach einem der vorherigen Ansprüche, wobei:
(a) die umlaufende Wand einen Wandabschnitt hat, der eine zumindest im Wesentlichen gerade Oberfläche hat, und/oder
(b) die umlaufende Wand eine zumindest im Wesentlichen zylindrische Form hat, und/oder
(c) der Einlass (i) benachbart zum Auslass angeordnet oder (ii) im Deckel umfasst ist, und/oder
(d) das Trennkompartiment eine longitudinale Achse hat, wobei das Trennkompartiment gegebenenfalls eine zumindest im Wesentlichen gleichbleibende maximale Breite entlang der longitudinalen Achse hat, und/oder
(e) die umlaufende Wand einen zumindest im Wesentlichen röhrenförmigen Teil der Trennkompartiment bestimmt, und/oder
(f) das Trennkompartiment in einem Gehäuse umfasst ist, und/oder
(g) die durch den Einlass des Trennkompartiments eingeleitete Flüssigkeit eine wässrige Flüssigkeit ist.

9. Verfahren nach einem der vorhergehenden Ansprüche, ausgeführt in Anwesenheit einer Gravitationskraft, weiterhin umfassend Anordnung des Trennkompartiments in solch einer Orientierung, dass der Auslass in Richtung der Gravitationskraft in einem Winkel von +75° bis -75° zeigt, bevor das Fluid, welches zumindest im Wesentlichen nicht mischbar mit der die Zellzellpopulation umgebenden Flüssigkeit ist, in das Trennkompartiment eingeleitet wird,
wobei das Trennkompartiment gegebenenfalls in solch einer Orientierung angeordnet ist, dass der Auslass zumindest im Wesentlichen in Richtung der Gravitationskraft zeigt, bevor das Fluid, welches zumindest im Wesentlichen nicht mischbar mit der die Zielzellpopulation umgebenden Flüssigkeit ist, in das Trennkompartiment eingeleitet wird.

10. Verfahren nach einem der vorherigen Ansprüche, wobei das Trennkompartiment eine longitudinale Achse hat und wobei der Auslass zumindest im Wesentlichen an der longitudinalen Achse angeordnet ist, wobei gegebenenfalls Anordnen des Trennkompartiments in solch einer Orientierung, dass der Auslass so ausgerichtet ist, dass er zumindest im Wesentlichen in oder entgegengesetzt der Richtung der Gravitationskraft zeigt, in Position Bringen des Trennkompartiments umfasst, in der die longitudinale Achse zumindest im Wesentlichen parallel zur Gravitationskraft ist.

11. Verfahren nach einem der vorherigen Ansprüche, wobei das Trennkompartiment einen einzelnen Einlass umfasst oder wobei das Trennkompartiment zwei Einlässe umfasst.

12. Verfahren nach Anspruch 11, wobei das Trennkompartiment zwei Einlässe umfasst und wobei ein Einlass benachbart zum Auslass angeordnet ist und wobei Einleiten eines zumindest im Wesentlichen konstanten Flusses einer geeigneten Flüssigkeit durch den Einlass des Trennkompartiments nach dem Anordnen der Trennkompartiment in solch einer Orientierung durchgeführt wird, dass der Auslass so positioniert wurde, sodass er in Richtung der Gravitationskraft zeigt.

13. Verfahren nach einem der vorherigen Ansprüche,
(a) wobei die Vielzahl von Zellen in zumindest in einer Körperflüssigkeitsprobe, einer Umweltprobe, einer Zellkulturprobe, einer Knochenmarksprobe, einer Abwasserprobe, einer Nahrungsmittelprobe, einer Milchprobe, einer forensischen Probe, einer Biomolekülproduktionsprobe, einer Proteinherstellungsprobe, einer Lipidherstellungsprobe, einer Kohlenhydratherstellungsprobe und in jeder möglichen Kombination davon enthalten ist,
wobei gegebenenfalls die Körperflüssigkeitsprobe eine Blutprobe, eine Serumprobe, eine amniotische Flüssigkeitsprobe, eine Samenflüssigkeitsprobe, eine lymphatische Flüssigkeitsprobe, eine zerebrospinale Flüssigkeitsprobe, eine Nasenrachenraumspülungsprobe, eine Sputumsprobe, eine Mundabstrichsprobe, eine Rachenabstrichsprobe, eine Nasenabstrichsprobe, eine bronchoalveolare Spülprobe, eine Bronchialsekretprobe oder eine Urinprobe ist; und/oder
(b) wobei die Zellzellpopulation eine Population somatischer Zellen, eine Population von Stammzellen oder eine Population von Vorläuferzellen, und/oder
wobei die Zielzellpopulation eine Population von Tumorzellen, fötalen Zellen und embryonischen Zellen ist.

14. Verfahren nach einem der Ansprüche 1 bis 3 und 6 bis 13, wobei Anordnen des Trennkompartiments in solch einer Orientierung, dass der Auslass mit einem Winkel von +75° bis -75° in Richtung der Gravitationskraft oder mit einem Winkel von +75° bis -75° in Gegenrichtung der Gravitationskraft zeigt, Rotieren der Trennkompartiment umfasst.

15. Verfahren nach einem der vorherigen Ansprüche, weiterhin umfassend:
(d) Sammeln der die Zielzellpopulation umgebende Flüssigkeit zusammen mit der Zielzellpopulation aus dem Trennkompartiment.

## Revendications

1. Procédé d'enrichissement et/ou d'isolation d'une population de cellules cibles à partir d'une pluralité de cellules en présence d'une force gravitationnelle, le procédé comportant:
(a) introduire la pluralité de cellules dans un compartiment de séparation,
le compartiment de séparation ayant une entrée et une sortie en communication de fluide avec l'ambiance, le compartiment de séparation étant défini par une paroi périphérique, une base et une partie supérieure,
la base et la partie supérieure étant agencée dans un rapport au moins sensiblement opposé dans le compartiment de séparation;
(b) dans un agencement de compartiment de séparation où la sortie est positionnée pour faire face dans un angle ± 75° de façon opposé à la direction de la force gravitationnelle, introduire un débit au moins sensiblement constant d'un liquide approprié par l'entrée du compartiment de séparation, et permettre à un débit de liquide au moins sensiblement constant de sortir le compartiment de séparation à travers la sortie en suspendant la pluralité de cellules dans un débit constant de liquide de manière que le débit constant de liquide empêche la population de cellules cibles de se sédimenter et de sortir le compartiment de séparation à travers la sortie;
et ainsi enrichir et/ou isoler la population de cellules cibles.

2. Procédé selon la revendication 1, la sortie comportant un médium poreux ayant des pores de plus petites tailles que la taille moyenne de cellules des cellules de la population de cellules cibles, le procédé comportant en outre:
(c) introduire un fluide dans le compartiment de séparation à travers de son entrée, le fluide étant au moins sensiblement immiscible avec le liquide entourant la population de cellules cibles dans le compartiment de séparation, et d'une densité inférieure à celui-ci, laisser le liquide entourant la population de cellules cibles sortir à travers la sortie de manière que le volume de celui-ci peut diminuer.

3. Procédé selon l'une quelconque des revendications précédentes:
(a) l'introduction d'un débit au moins sensiblement constant d'un liquide approprié à travers l'entrée du compartiment de séparation étant effectuée après agencement du compartiment de séparation dans une telle orientation que la sortie est positionnée pour faire face au moins sensiblement de façon opposé à la direction de la force gravitationnelle, et/ou
(b) la sortie étant compris dans la base ou dans la partie supérieure, et/ou
(c) l'entrée et la sortie étant agencée dans un rapport au moins sensiblement opposé l'un à l'autre.

4. Procédé d'enrichissement et/ou d'isolation d'une population de cellules cibles à partir d'une pluralité de cellules, le procédé comportant:
(a) introduire la pluralité de cellules dans un compartiment de séparation,
le compartiment de séparation ayant une entrée et une sortie en communication de fluide avec l'ambiance, le compartiment de séparation étant défini par une paroi périphérique, une base et une partie supérieure,
la base et la partie supérieure étant agencée dans un rapport au moins sensiblement opposé dans le compartiment de séparation, la base comportant la sortie, et la sortie comportant un médium poreux ayant des pores de plus petites tailles que la taille moyenne de cellules des cellules de la population de cellules cibles;
(b) introduire un débit au moins sensiblement constant d'un liquide approprié par l'entrée du compartiment de séparation, et permettre à un débit de liquide au moins sensiblement constant de sortir le compartiment de séparation à travers la sortie en suspendant la pluralité de cellules dans un débit constant de liquide de manière que le débit constant de liquide empêche la population de cellules cibles de se sédimenter et le médium poreux empêche la population de cellules cibles de sortir le compartiment de séparation à travers la sortie;
et ainsi enrichir et/ou isoler la population de cellules cibles.

5. Procédé selon la revendication 4, comportant en outre:
(c) introduire un fluide dans le compartiment de séparation par son entrée, le fluide étant au moins sensiblement immiscible avec le liquide entourant la population de cellules cibles dans le compartiment de séparation, et d'une densité inférieure à celui-ci, laisser le liquide entourant la population de cellules cibles sortir à travers la sortie de manière que le volume de celui-ci peut diminuer.

6. Procédé selon la revendication 4 ou 5,
(a) le procédé étant effectué en présence d'une force gravitationnelle, l'introduction d'un débit au moins sensiblement constant d'un liquide approprié par l'entrée du compartiment de séparation étant effectuée après agencement du compartiment de séparation dans une telle orientation que la sortie est positionnée pour faire face dans un angle de ± 75° à la direction de la force gravitationnelle après l'introduction de la pluralité de cellules dans le compartiment de séparation, et/ou
(b) le compartiment de séparation étant agencé dans une telle orientation que la sortie est positionnée pour faire face au moins sensiblement dans la direction de la force gravitationnelle alors que le débit au moins sensiblement constant d'un liquide approprié est introduit par l'entrée du compartiment de séparation.

7. Procédé selon l'une quelconque des revendications précédentes:
(a) le procédé étant effectué en présence d'une force gravitationnelle, le débit au moins sensiblement constant de liquide introduit par l'entrée du compartiment de séparation étant permis de créer une force sur la population de cellules cibles qui s'oppose à la force gravitationnelle, et/ou
(b) le fluide introduit dans le compartiment de séparation étant un gaz, et/ou le fluide introduit dans le compartiment de séparation étant de l'air, et/ou
(c) le procédé comportant en outre l'inclinaison du compartiment de séparation par rapport à son orientation initiale après avoir introduite la pluralité de cellules dans le compartiment de séparation, et/ou
(d) l'enrichissement de la population de cellules cibles étant au moins 50 fois plus, 75 fois plus, 100 fois plus, 150 fois plus ou 200 fois plus, et/ou
(e) le procédé comportant en outre l'agitation du compartiment de séparation après que celui-ci a été agencé dans une orientation où la la sortie est positionnée pour faire face au moins sensiblement dans la direction de la force gravitationnelle.

8. Procédé selon l'une quelconque des revendications précédentes:
(a) la paroi périphérique ayant une partie de paroi qui a une surface sensiblement droite, et/ou
(b) la paroi périphérique ayant une forme sensiblement cylindrique, et/ou
(c) l'entrée étant (i) agencée à proximité de la sortie ou (ii) compris dans la partie supérieure, et/ou
(d) le compartiment de séparation ayant un axe longitudinal, le compartiment de séparation ayant facultativement une largeur maximum au moins sensiblement uniforme le long de l'axe longitudinal, et/ou
(e) la paroi périphérique définissant une partie au moins sensiblement tubulaire du compartiment de séparation, et/ou
(f) le compartiment de séparation étant compris dans un boîtier, et/ou
(g) le liquide introduit dans l'entrée du compartiment de séparation étant un liquide aqueux.

9. Procédé selon l'une quelconque des revendications précédentes étant effectué en présence d'une force gravitationnelle, le procédé comportant en outre l'agencement du compartiment de séparation dans une telle orientation que la sortie est positionnée pour faire face dans un angle de +75° à -75° de la force gravitationnelle avant que le fluide qui est au moins sensiblement immiscible avec le liquide entourant la population de cellules cibles soit introduit dans le compartiment de séparation,
le compartiment de séparation étant facultativement agencé dans une telle orientation que la sortie est positionnée pour faire face au moins sensiblement dans la direction de la force gravitationnelle avant que le fluide qui est au moins sensiblement immiscible avec le liquide entourant la population de cellules cibles soit introduit dans le compartiment de séparation.

10. Procédé selon l'une quelconque des revendications précédentes, le compartiment de séparation ayant un axe longitudinal, et la sortie étant agencée au moins sensiblement à l'axe longitudinal, l'agencement facultatif du compartiment de séparation dans une telle orientation que la sortie est positionnée pour faire face au moins sensiblement dans la direction ou de façon opposé à la direction de la force gravitationnelle comportant la mise du compartiment de séparation dans une position où l'axe longitudinal est au moins sensiblement en parallèle à la force gravitationnelle.

11. Procédé selon l'une quelconque des revendications précédentes, le compartiment de séparation comportant une entrée unique ou le compartiment de séparation comportant deux entrées.

12. Procédé selon la revendication 11, le compartiment de séparation comportant deux entrées et une entrée étant agencée à proximité de la sortie et l'introduction d'un débit au moins sensiblement constant d'un liquide approprié par l'entrée du compartiment de séparation étant effectuée après l'agencement du compartiment de séparation dans une telle orientation que la sortie est positionnée pour faire face au moins sensiblement dans la direction de la force gravitationnelle.

13. Procédé selon l'une quelconque des revendications précédentes,
(a) la pluralité de cellules étant comprise dans au moins un d'un échantillon de fluide corporel, un échantillon environnemental, un échantillon de culture cellulaire, un échantillon de moelle osseuse, un échantillon d'eaux usées, un échantillon alimentaire, un échantillon de lait, un échantillon médico-légal, un échantillon de production de molécule biologique, un échantillon de préparation de protéine, un échantillon de préparation de lipide, un échantillon de préparation de carbohydrate, et toute combinaison de ceux-ci,
l'échantillon de fluide corporel étant facultativement un d'un échantillon de sang, un échantillon de sérum, un échantillon de liquide amniotique, un échantillon de semence, un échantillon de fluide lymphatique, un échantillon de fluide cérébrospinal, un échantillon de lavage nasopharyngé, un échantillon de crachat, un échantillon de prélèvement de la muqueuse buccale, un échantillon de prélèvement de la gorge, un échantillon de prélèvement nasal, un échantillon de lavage bronchoalvéolaire, un échantillon de sécrétion bronchique et un échantillon d'urine; et/ou
(b) la population de cellules cibles étant une population de cellules somatiques, une population de cellules souches ou une population de cellules progénitrices, et/ou
la population de cellules cibles étant une population de l'un des cellules tumorales, des cellules foetales et des cellules embryonnaires.

14. Procédé selon l'une quelconque des revendications 1 à 3 et 6 à 13, l'agencement du compartiment de séparation dans une telle orientation que la sortie est positionnée pour faire face dans un angle de +75° à -75° dans la direction de la force gravitationnelle ou dans un angle de +75° à -75° de façon opposé à la direction de la force gravitationnelle comportant la rotation du compartiment de séparation.

15. Procédé selon l'une quelconque des revendications, comportant:
(d) recueillir le liquide entourant la population de cellules cibles ensemble avec la population de cellules cibles issue du compartiment de séparation.
